# EUROPEAN PATENT APPLICATION

(11) **EP 4 821 543 A2**
(43) Date of publication of application: **14.10.2026**
(21) Application number: 26199010.5
(22) Date of filing: 29.11.2023
(51) Int. Cl.: A61N 1/44

(54) **TECHNOLOGIES FOR PLASMA PERIODONTAL TREATMENT DEVICES AND METHODS**

(30) Priority: 30.11.2022 US 202263428837 P
(62) Document number(s) of the earlier application(s) in accordance with Art. 76 EPC: 23829264.3 / 4 626 358
(71) Applicant: Colgate-Palmolive Company, New York, NY 10022-7499 (US); Nova Plasma Ltd., 1923000 D.N. Hevel Megiddo (IL)
(72) Inventor: LAM, Amnon, 1922500 Kibbutz Givat Oz (IL); HARHOL, Aviad, 4720908 Ramat Hasharon (IL); GARCIA GODOY SOCIAS, Bayardo Jose, Princeton, 08540 (US); GUIRGUIS, Mark, Scotch Plains, 07076 (US); Steele, Joseph Allan McKinnon, Plainfield, 07060 (US); DU-THUMM, Laurence, Princeton, 08540 (US)
(74) Representative: Sonnenhauser, Thomas Martin

(57) **Abstract**

A hand-held dental device may be configured to generate plasma for treatment of periodontal disease in a targeted tissue in a subject's oral cavity. The device may comprise a tip configured to apply a plasma generating electro-magnetic field. The tip comprises an electrically conductive tube extending from a proximal end of the tube to a distal end of the tube and having at least one hole near the distal end of the tube. The device may comprise at least one electrode disposed within the electrically conductive tube in at least one orientation with the electrically conductive tube. The disposition arranged to provide an air gap between the electrically conductive tube and the at least one electrode at least along a portion of the electrically conductive tube. The at least one electrode comprises at least one layer of insulation completely covering the at least one electrode.

## Description

### CROSS-REFERENCE TO RELATED APPLICATIONS

This application claims the benefit of U.S. Provisional Patent Application No. 63/428,837, filed on November 30, 2022, the entire contents of which are incorporated by reference herein, for all purposes.

### BACKGROUND

Periodontal disease, or periodontitis, may be commonly referred to as gum disease. Periodontitis is a gum infection that damages the soft tissue in the oral cavity. Without treatment, periodontitis can destroy the bone that supports dentition (e.g., teeth). Also, periodontitis can cause teeth to loosen, may lead to tooth loss, and/or may cause bleeding gums, among other dental issues.

Plasma treatments may be performed by a periodontist, a dentist, and/or a dental hygienist. Treatment may include thorough cleaning of the periodontal pockets around teeth to prevent damage to the surrounding bone. Treatment may include scaling, root planing, and/or antibiotics. Treatment may include pocket reduction surgery, soft tissue grafts, bone grafting, guided tissue regeneration, and/or tissue-stimulating proteins.

### BRIEF SUMMARY

Technologies are disclosed for a dental device configured to generate plasma for treatment of periodontal disease in a targeted tissue in a subject's oral cavity. In some embodiments the device may be hand-held. In some embodiments the device may have a handpiece connected to a desk-top base-station. The device may comprise a tip configured to apply a plasma generating electro-magnetic field. The tip may comprise an electrically conductive tube. The tube may extend from a proximal end of the tube to a distal end of the tube. In some embodiments the electrically conductive tube may be coated by a dielectric layer serving as an electric insulation. The dielectric layer may be applied on the internal surface of the tube or on the external surface of the tube or on the edges of the tube or on combinations thereof or on portions thereof. The electrically conductive tube may be in electrical communication with a device electrical ground reference point, thereby having a zero voltage or a low voltage relative to the device electrical ground during operation. The device may comprise at least one electrode. The at least one electrode may be disposed within the electrically conductive tube in one or more orientations (e.g., at least a partially coaxial and/or at least a partially concentric, etc., among others) within the electrically conductive tube. The coaxial disposition may be arranged to provide an air gap between the electrically conductive tube and the at least one electrode along at least portions of the tube. The at least one electrode is electrically insulated from the electrically conductive tube, for which end it may comprise in some embodiments at least one layer of insulation. The device may comprise a source of pressurized gas. The pressurized gas may provide a plasma source when subjected to a plasma-generating electro-magnetic (EM) field. Plasma may be generated at the device' tip upon an application of an electro-magnetic (EM) field between the at least one electrode and the electrically conductive tube, e.g., by supplying high voltage (HV) to the at least one electrode relative to the electrically conductive tube.

The dental device may further comprise a control unit. The control unit may comprise a memory, a communication interface, and/or a processor. In some embodiments the processor may be configured to monitor a pressure measurement inside the subject's oral cavity. The processor may be configured to compare the pressure measurement to a predetermined pressure value. In some embodiments the processor may compare the pressure measurement to the ambient pressure. In some embodiments the processor may be configured to cause the plasma generation to stop upon the pressure measurement exceeding the predetermined value. In some embodiments the processor may be configured to permit the plasma generation to continue upon the pressure measurement being substantially equal to the predetermined pressure value, or less than the predetermined pressure value.

### BRIEF DESCRIPTION OF DRAWINGS

The elements and other features, advantages and disclosures contained herein, and the manner of attaining them, will become apparent and the present disclosure will be better understood by reference to the following description of various examples of the present disclosure taken in conjunction with the accompanying drawings, wherein:
FIG. 1A is a diagram that schematically depicts an example of a periodontal treatment device.
FIG. 1B is a diagram that schematically depicts an example of a periodontal treatment device.
FIG. 2A is a diagram that schematically depicts a cross-section of an example embodiment of a handpiece for plasma treatment of a periodontal pocket.
FIG. 2B depicts schematically an example detailed view of distal end/part of a handpiece for plasma treatment of a periodontal pocket.
FIG. 2C depicts an enlarged view of tip distal portion/end of a handpiece for plasma treatment of a periodontal pocket.
FIG. 2D illustrates an example schematic of a cross-section of an example of a handpiece for plasma treatment of a periodontal pocket.
FIG. 3A illustrates an example schematic of an electric circuit configured to sample the anode current at the tip for a device for plasma treatment of a periodontal pocket.
FIG. 3B illustrates an example schematic of an electric circuit configured to sample the anode current at the tip for a device for plasma treatment of a periodontal pocket.
FIG. 4A illustrates an example schematic of a tip comprising a thermocouple for a device for plasma treatment of a periodontal pocket.
FIG. 4B illustrates an example schematic of a periodontal treatment device for plasma treatment of a periodontal pocket.
FIG. 5A illustrates an example schematic of a tip configured to produce plasma in an arcing mode for a device for plasma treatment of a periodontal pocket.
FIG. 5B illustrates an example schematic of a tip configured to produce plasma in an arcing mode for a device for plasma treatment of a periodontal pocket.
FIG. 6A depicts schematically a perspective of an example of a dental suction device comprising an active filter for absorbing ozone.
FIG. 6B depicts a cross section of an example suction head and active filter of a dental suction device.
FIG. 6C schematically depicts an example of a distal part of a handpiece of a plasma-assisted periodontal treatment device.
FIG. 7A illustrates an example twisted electrode that may be used with one or more periodontal treatment devices.
FIG. 7B illustrates an example configuration of a twisted electrode disposed inside a metallic tube that may be used with one or more periodontal treatment devices.
FIG. 7C illustrates an example of a single wire electrode which is manufacturable from a piece of insulated wire cut from a wire roll.
FIG. 8A illustrates an isometric view of an example distal end/part that may be used with one or more plasma generating treatment devices.
FIG. 8B illustrates a front view of an example distal end/part that may be used with one or more plasma generating treatment devices.
FIG. 8C illustrates a side view of an example distal end/part that may be used with one or more plasma generating treatment devices.
FIG. 8D illustrates a top view of an example distal end/part that may be used with one or more plasma generating treatment devices.
FIG. 9 illustrates an example characteristic of a flow rate of efficacious nitric oxide (NO) output as compared to airflow for one or more of the devices described herein.
FIG. 10 is a block diagram of a hardware configuration of an example device that may control one or more plasma generating devices described herein.

### DETAILED DESCRIPTION

For the purposes of promoting an understanding of the principles of the present disclosure, reference will now be made to the examples illustrated in the drawings, and specific language will be used to describe the same. It will nevertheless be understood that no limitation of the scope of this disclosure is thereby intended.

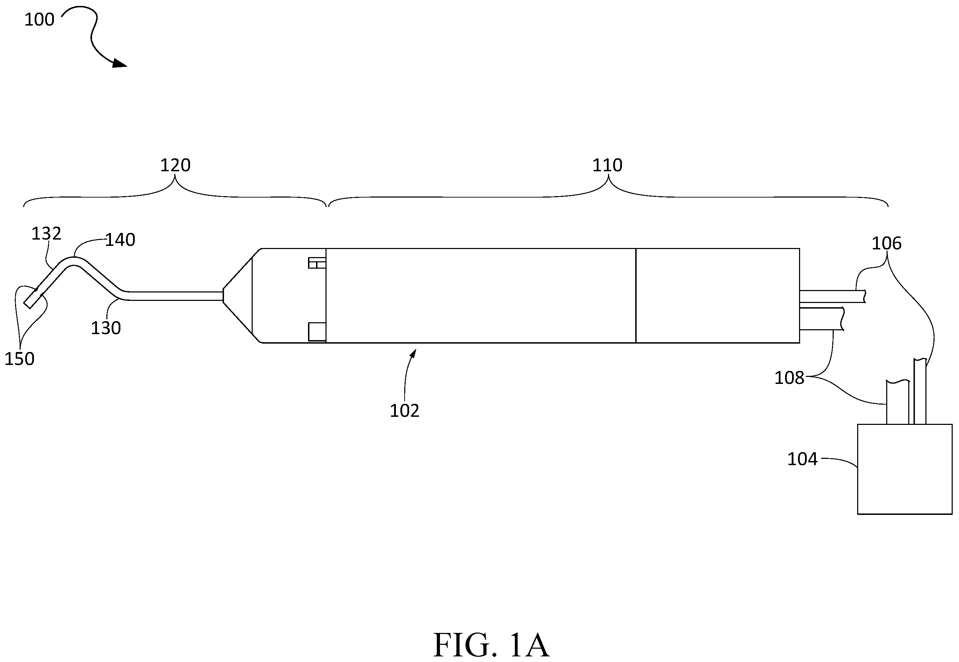

FIG. 1A is a diagram that schematically depicts an example embodiment of a periodontal treatment device 100. Device 100 is adapted and configured to generate plasma inside a periodontal pocket, to disinfect the pocket interior, and to assist in accelerated healing of a lesion - such as wound, abscess, tumor or the like - that might be present in or around the pocket.

Device 100 comprises a handpiece 102 and an operational unit 104, associated to one another by an electric cord 106 and a gas tube 108. The operational unit 104 may be configured as a desk-top station, a portable station, and/or a distributed control unit, among other configurations, for example. Handpiece 102 comprises a proximal part 110 and a distal part 120. Proximal part 110 is configured to allow a medical practitioner such as a dentist to hold and manipulate handpiece 102 by hand. Distal part 120 comprises a plasma-generating tip 130. Tip 130 is configured elongated and narrow so as to allow penetrating a tip distal portion 132 thereof into a periodontal pocket. Typically, tip 130 may be a few centimeters long. In some embodiments tip 130 is between 1cm and 10 cm long, in some scenarios between 3 cm and 7 cm long. Tip distal portion 132 may have an external diameter smaller than 2 mm, in some scenarios smaller than 1.5mm, in some scenarios a diameter of 1mm or less. To allow insertion of the tip distal portion into the periodontal pocket, among other reasons, the distal end has the above-mentioned external diameter along a distance of at least 0.5cm, in some scenarios along a distance of at least 1cm, in some scenarios along a distance of 2 cm or more. In one or more scenarios, for example, tip 130 may be curved as depicted in FIG. 1A. In one or more scenarios, for example, tip 130 may be configured straight.

Tip 130 may generate plasma by applying, at least at the tip distal portion 132, a plasma-generating electromagnetic (EM) field between a cathode and an anode, as is explained in greater detail below. The cathode comprises an electrically conducting hollow tube 140 which may be electrically connected to the device ground potential. In one or more scenarios, the cathode may be metallic. The anode comprises an elongated conductor (not shown) extending substantially within (e.g., coaxially, concentrically, etc.) along the hollow tube there inside. The anode is electrically isolated from the cathode by an insulating layer (not shown) between the anode and the cathode. In some embodiments the anode may be coated by the insulating layer. In one or more scenarios, the internal surface of the cathode's hollow tube may be thus coated.

High voltage (HV) may be supplied to the anode, thereby effecting a plasma generating EM field between the anode and the cathode. The HV is typically alternating (AC), referred to herein as radio-frequency (RF). AC voltage may be at a frequency lower than 10MH, typically between 3KHz and 5MHz, in some scenarios between 30KHz and 600KHz. In some embodiments, the HV may be modulated ON-OFF, so as to enable simple control over the average power delivered for generating plasma, by controlling the duty cycle of the modulated HV.

The hollow tube of the cathode may be grounded, and/or may serve to prevent arcing from the anode to the treated region of the patient (e.g., the periodontal pocket - not shown). Electrically associating the hollow tube to any well-defined, low voltage relative to the high voltage which is supplied to the anode, may be sufficient to protect from arcing between the anode and the treated region outside the hollow tube. For example, the hollow tube may be referred to herein as 'grounded' for purposes of illustration. Further, 'low voltage relative to the high voltage which is supplied to the anode' may refer to a voltage low enough such that may not cause arcing.

In one or more scenarios, employing variable duty cycle to control the average power delivered to the plasma may have advantages (e.g., perhaps in addition to simplicity of power control, etc.). The dielectric strength of many/most dielectric materials (e.g., many/most polymers in particular) may decrease with the increase of temperature. In one or more scenarios, perhaps as the average power increases, the risk of electric breakdown in the insulation between the anode and the cathode, may increase. It may be useful to control the average power by varying the duty cycle (e.g., at a fixed and/or relatively low HV) perhaps than to vary the HV itself, among other scenarios.

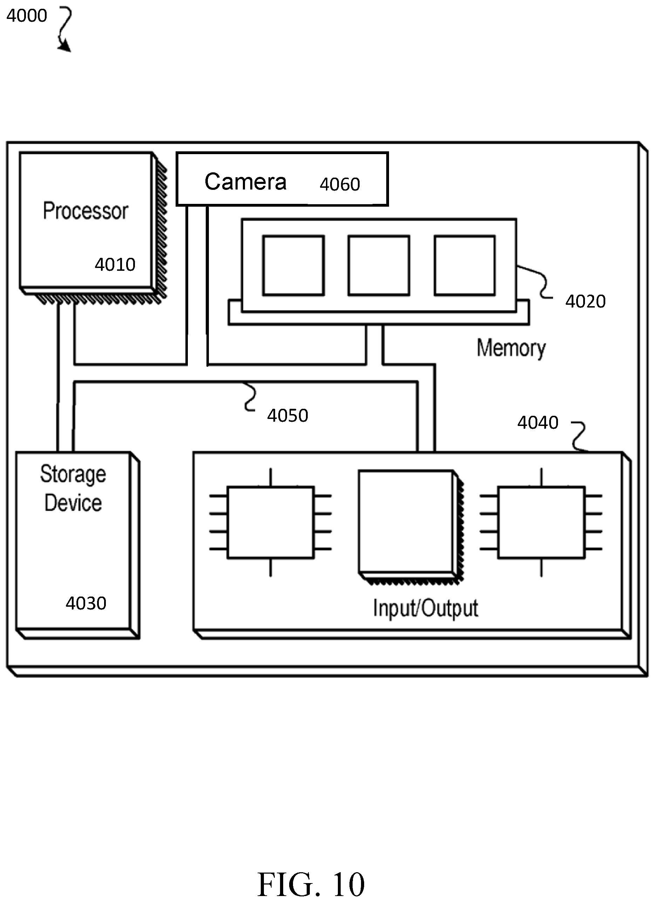

In one or more scenarios, automatic control of the plasma average power may be employed. A desired average power may be set according to one or more plasma-related parameters that may be sensed and/or measured during operation (e.g., among other scenarios/times). For example, a local temperature may be measured at and/or near where plasma is generated. The temperature reading may be supplied to a controller and/or a processor(s) of the device (e.g., further described in regard to FIG. 10). Perhaps according to the temperature reading and/or upon a comparison of the temperature reading to pre-determined threshold and/or a temperature scale and/or following a pre-programed routine, the controller and/or processor may affect a variation of the duty cycle. For example, a decrease of the duty cycle, and/or a decrease of average power, may be affected in response to a temperature reading that is higher than a pre-determined value, among other scenarios.

The duty cycle may be set to any value(s) in the range 0-100%, for example in the range 0.1-90%. The selected duty cycle and/or the modulation frequency may be (e.g., advantageously) selected in consideration with the radio-frequency (RF) of the HV, so that at least several cycles of the RF HV could be included in the "ON" time of the modulation. For example, at a HV frequency of 100KHz and/or a modulation frequency of 100Hz, a duty cycle of 1%, the "ON" is 100microseconds (usec), perhaps for example allowing for about 10 cycles of the HV during one or more, or each modulation cycle. If, relative to the described working point, a further decrease of average power may be useful, and/or assuming the HV frequency may not be changed, among other scenarios, it may be useful to reduce the modulation frequency to enable a reduction of the duty cycle of the HV, perhaps without reducing the number of HV cycles during each "ON" period, for example.

Electric power for generating the plasma is delivered from the operational unit 104 via electric cord 106, whereas operational unit 104 may receive electric power from an external source (not shown) such as a wall outlet for example or from a battery. In one or more scenarios, operational unit 104 delivers to the handpiece 102 electric power at low voltage, whereas a HV transformer in the handpiece 102 transforms the low voltage to high voltage, as is further described herein. In some embodiments, operational unit 104 may generate the HV required to generate the plasma, and the HV is delivered from the operational unit directly to plasma generating tip 130 whereas electric cord 106 is a HV electric cord. In some embodiments no voltage manipulation or transformation is carried out in operational unit 104, in other words all voltage manipulation and transformation required to generate the HV is carried out entirely within the handpiece 102.

In one or more scenarios, perhaps for example when the device is activated, among other scenarios, the generation of plasma through the plasma arcs and/or the HV transformer activation may create an audible noise. This noise can be picked up through a microphone. A microphone may also pick up other noises in the surrounding area. In one or more scenarios, an accelerometer and/or an inertial measurement unit (IMU) can be used to pick up the vibrations in the handle that may originate from the audible noise. This has the added usefulness of not being vulnerable to sensing the surrounding audible noise. Such a sensor may be placed inside the handle near the HV transformer (e.g., of any of the devices described herein). The accelerometer/IMU (not shown) may sense the hand motions of the clinician manipulating the handle during a procedure. These motions may be at a low frequency, perhaps as compared to the plasma related vibrations. A high pass and/or bandpass filter can be employed to capture (e.g., only) the vibration frequency of interest as it pertains to plasma generation. Once identified, the amplitude and/or frequency fluctuations of the vibrations can be characterized to correlate with an intensity of plasma generation during operation. One or more operational and/or safety interlocks regarding plasma generation, gas pressurization, HV generation, and/or EM field generation, or the like, may be based on an output/detection of the accelerometer/IMU, for example.

The internal diameter of the hollow tube 140 and the external diameter of the elongated conductor of the anode may be dimensioned so as to leave, at least along portions of the hollow tube, an air gap (not shown here) therebetween. The air gap may allow for a space between the anode and the cathode where plasma is generated, thereby generating gaseous radicals and active species that take part in the medical effects of the plasma in the periodontal pocket. Furthermore, the gap enables the hollow tube to be employed as a channel for flowing the generated plasma out from the hollow tube via holes or openings 150 in the tube at the tip distal portion 132. One or more holes 150 may be dimensioned so as to allow, on the one hand, sufficient gas flow throughout, for example, plasma delivery through the holes towards the treated region, and/or on the other hand to maintain and/or ensure sufficient EM shielding of the anode by the cathode to prevent arcing from the anode to the treated region. Gas flow through the hollow tube may be forced by a gas pump (not shown here) located in operational unit 104, via gas tube 108. Tip distal portion 132 may have a hole only at the distal end of the tube. In other embodiments the distal end of the tube may be sealed and the holes may be located on sides of the tube.

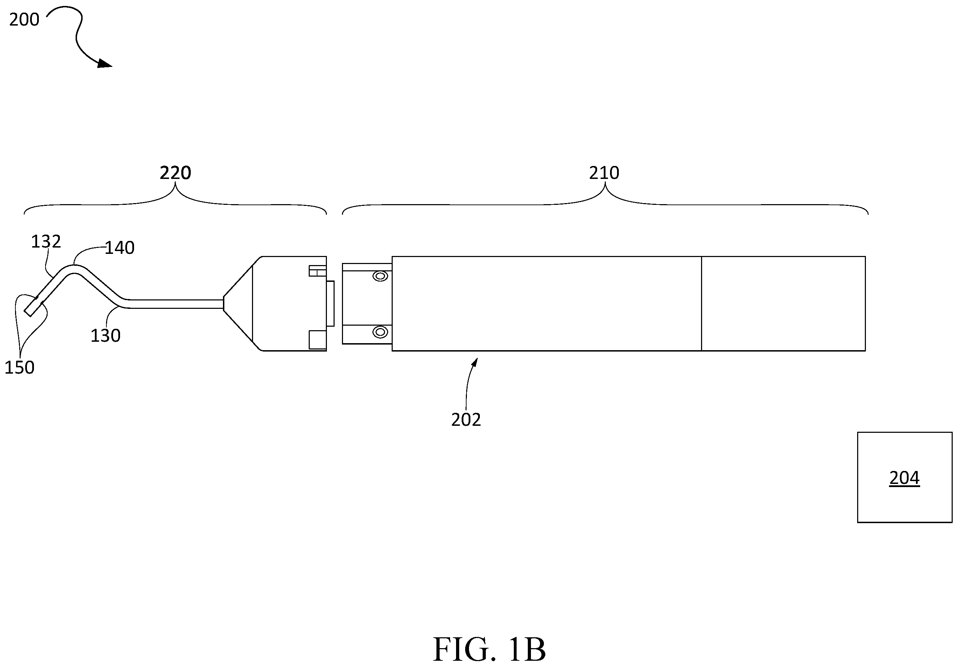

FIG. 1B schematically depicts an example embodiment of a periodontal treatment device 200. Like device 100, at least in part, device 200 may be adapted and/or configured to generate plasma inside a periodontal pocket (not shown). Device 200 comprises a handpiece 202 and an operational unit 204. Handpiece 202 is a stand-alone unit having an autonomous energy source (not shown here) such as a battery. Respectively, operational unit 204 may be used as a docking station and charger for the handpiece 202.

For generating gas flow through the tip 130, among other reasons, handpiece 202 comprises a gas pump (not shown). For generating plasma, handpiece 202 comprises at least a HV transformer. The gas pump and the HV transformer may receive energy from the autonomous energy source (e.g., the battery) of the handpiece 202.

Handpiece 202 may comprise a proximal part 210 and a distal part 220 comprising the plasma-generating tip 130. In one or more scenarios, the proximal part houses the autonomous energy source, the gas pump and the HV transformer. Distal part 220 is easily detachable from proximal part 210, so as to allow a practitioner to replace the distal part and the tip 130 after each treatment and use a new tip for each patient.

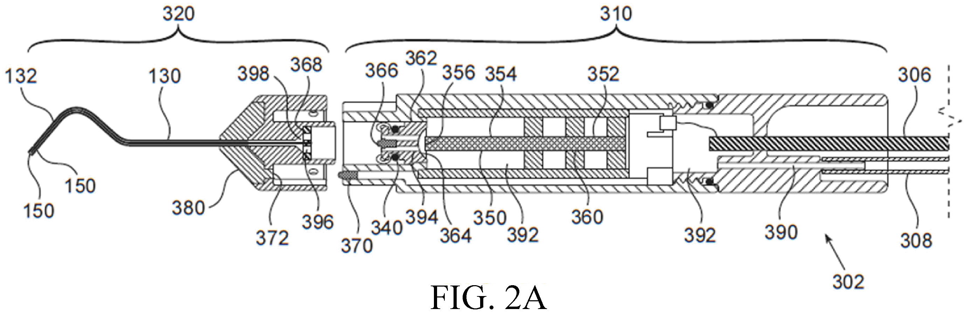

FIG. 2A schematically depicts a cross-section of an example embodiment of a handpiece 302 for plasma treatment of a periodontal pocket. Handpiece 302 comprises a proximal part 310 configured to be associated with an operational unit (not shown here) via an electric cord 306 and a gas tube 308. Proximal part 310 comprises a piezoelectric (PE) HV transformer 350, for transforming a RF low voltage signal to a RF high voltage which is supplied to the device' anode as is further detailed below. Handpiece 302 further comprises a detachable distal part 320, comprising plasma-generating tip 130.

Piezoelectric (PE) transformer 350 is an embodiment of a resonance transformer, which is based on piezoelectricity and forms an electromechanical system. For example, piezoelectric transformer 350 may be a Rosen-type transformer. Alternatively, other types of piezoelectric transformers can be used.

PE transformer 350 is constructed as an elongated rectangular cuboid (an elongated orthogonal parallelepiped) having, along its long dimension, a first region 352 that is an input region and a second region 354 that is an output region, comprising an output end 356. In the first region 352, the piezoelectric transformer 350 comprises electrodes 360 to which an alternating low voltage can be applied. The electrodes 360 have an internal portion inside the piezoelectric transformer 350 between layers of piezoelectric material, and an external portion, on opposite sides of the cuboid, to which the alternating low voltage is applied.

Due to the piezoelectric effect of the piezoelectric material, the alternating voltage applied on the input side is converted into a mechanical oscillation. The mechanical oscillation in turn, transforms back to electrical voltage at the output region 354 of the PE transformer. The frequency (e.g., a resonance frequency) of the mechanical oscillation depends essentially on the geometry, the mechanical structure and the material of the piezoelectric transformer 350. At resonance frequency, a high electrical voltage is generated between the output-side end 356 and the electrodes 360 of the first region 352.

The low voltage signal which is supplied to the electrodes 360 of the PE transformer may be generated by a driving signal generator (not shown) in the operational unit, and/or supplied to the handpiece via electric cord 306. In one or more scenarios, the low voltage signal may be of a few tens of volts, e.g., between 10 and 100V, in some scenarios between 20 and 60V, although voltages lower than 10V or higher than 100V may also be employed in some embodiments. The frequency of the low voltage signal is dependent on the PE transformer characteristics as explained above and is typically fine-tuned to fit to a piezoelectric resonance frequency of the PE transformer. In some embodiments the resonance frequency may be between 10KHz and 200KHz, although in some embodiments PE transformers having resonance frequencies outside this range may be used. In some embodiments the frequency of the low voltage signal is being optimized continuously and/or automatically by the driving signal generator, by monitoring and maximizing (or minimizing) a parameter of the low voltage signal (e.g., current, voltage, power, and/or phase, etc.) or a parameter of the high voltage signal, sampled by a sampling circuit as described further below.

In one or more scenarios, frequency of the low voltage signal may be optimized (continuously and/or automatically) by monitoring a plasma parameter (e.g., glow intensity, discharge current) in the handpiece and employing a feedback signal from the handpiece to the operational unit via electric cord 306. In some embodiments the low voltage signal may be modulated ON-OFF, so as to enable simple control over the average power delivered for generating plasma, by controlling the duty cycle of the modulated generated HV.

As described herein, operation of the PE transformer involves mechanical oscillations that cause the output end 356 to vibrate (typically at an ultrasonic frequency) at an amplitude that might reach 1mm or more. Consequently, it is typically favorable to induce the high voltage generated by the PE transformer in a nearby electrode without any Galvanic connection between the nearby electrode and the PE transformer. A cup electrode 362 has a concave face 364 facing the PE transformer and located at a short distance of a few millimeters or even less than one millimeter from the output end 356 of the PE transformer. During operation of the PE transformer, the high potential at the output end 356 ionizes the atmosphere around the output end, which renders the atmosphere between the output end 356 and the concave face 364 conductive, hence inducing the high potential of the output end in the cup electrode 362. It is noted that such ionization of the atmosphere around the output end is typically accompanied with a visible glow and generation of intermediate plasma in that space.

Cup electrode 362 comprises a HV pogo-pin (e.g., spring-loaded pin) 366, positioned on the side of cup electrode 362 opposite to concave face 364, and configured to contact an anode contact 368 of distal part 320, when the distal part is attached to proximal part 310. Proximal part 310 further comprises a ground pogo pin 370, connected to ground potential in the proximal part. Ground potential is supplied by the operational unit via the electric cord 306. Perhaps when the distal part is attached to proximal part 310, among other scenarios, ground pogo pin 370 may contact a ground contact 372 in distal part 320.

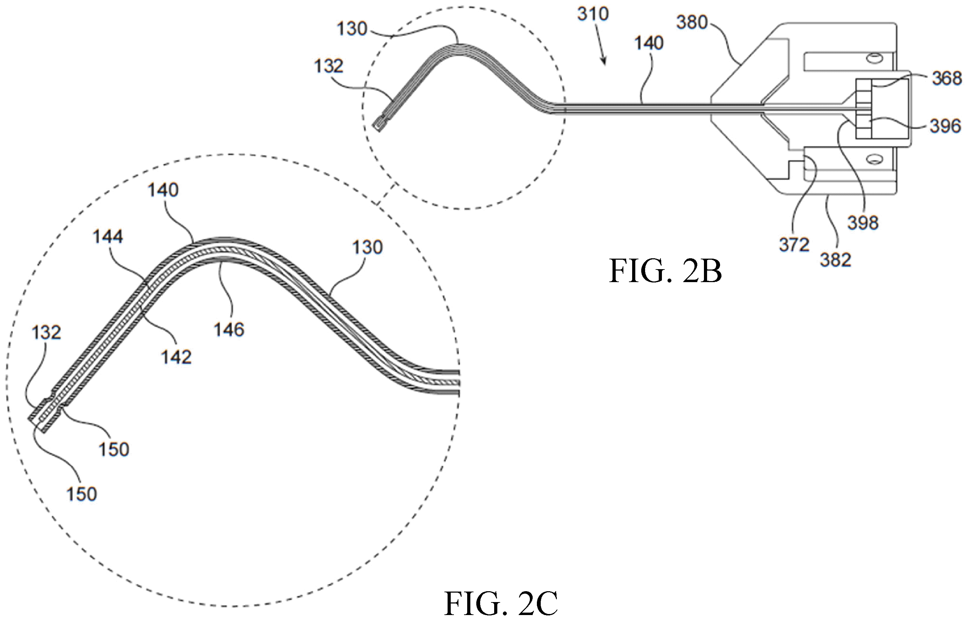

Referring to FIG. 2B and FIG. 2C that depict schematically an example detailed view of distal part 310 and an enlarged view of tip distal portion 132, respectively. Hollow tube 140 is firmly connected to metallic cone 380 of distal part 310, which is, in turn, electrically connected to ground contact 372. Hollow tube 140 may be manufactured from a high-strength, flexible and inert metal, e.g., stainless steel. Inside hollow tube 140, roughly concentrically, is arranged an anode 142, extending between anode contact 368 and tip distal portion 132. Anode 142 comprises an anode conductor 144 that may be insulated by an insulation layer 146 for electrical insolation between hollow tube 140 and anode conductor 144. Anode conductor 144 may be electrically connected to anode contact 368, hence having the same electric potential. Metallic cone 380, anode contact 368 and ground contact 372 are held together by a distal body member 382, made of a dielectric material, thereby ensuring electrical insulation between the anode conductor 144 and the anode contact 368 which are at anode potential during operation, and the metallic cone 380 and ground contact 372 which are at ground potential during operation.

Anode conductor 144 may be configured (e.g., as a flexible wire) and/or may be manufactured from high-strength, flexible and inert metal, e.g., stainless steel. insulation layer 146 may be made of a dielectric material, e.g., ceramic or polymeric coating. In some embodiments a dielectric coating may be made of Ethylene tetrafluoroethylene (ETFE) or Polytetrafluoroethylene (PTFE, or Teflon^{®}) or Parylene and/or or a combination thereof by way of concentric layers around anode conductor 144. The insulation layer thickness may be in the range between 10micrometers(um) and 800um, more preferably between 30 um and 500 um, even more preferably between 50 um and 200 um. In regions where hollow tube 140 is curved, as around curve 148, among other scenarios, insulation may be thicker compared to regions where the hollow tube is straight, to prevent electrical breakdown of the insulation. In some embodiments, insulation thickness in such curved regions may be doubled.

Returning to FIG. 2A, handpiece 302 is configured to enable a gas flow from gas tube 308 towards tip 130. During operation, gas may be forced by the operational unit via gas tube 308 into hollow gas channel 390 in proximal part 310. The gas may be air which is driven into the gas tube 308 by a gas pump (compressor) in the operational unit. In one or more scenarios, the gas may be stored in a pressurized gas reservoir and released controllably into the gas tube. One or more gases, for example different from air, may be employed, e.g., nitrogen, helium or argon, or a mixture thereof, among others.

Inside Proximal part 310, the gas proceeds under pressure from gas channel 390 to internal gallery 392 around PE transformer 350, and into cap electrode channels 394. Cup electrode channels 394 are arranged circumferentially around pogo-pin 366 and lead the gas to anode contact perforations 396 in anode contact 368 in distal part 320 (FIG. 2B). The gas that passes through anode contact perforations 396 is collected by funnel construction 398 in distal body member 382 and is flown on into hollow tube 140. The gas is eventually released via openings 150 in tip distal portion 132. A seal 340 between proximal part 310 and distal part 320 may prevent the gas from escaping therebetween.

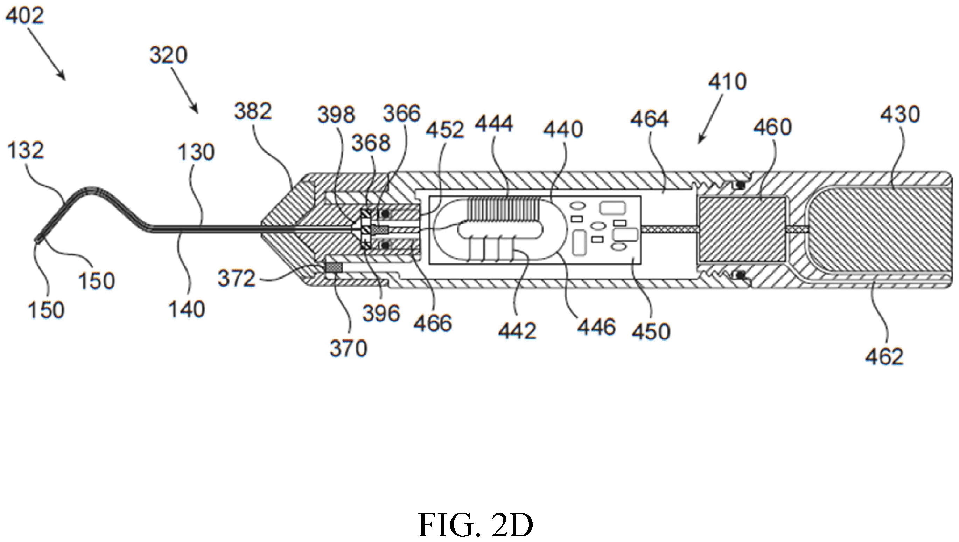

FIG. 2D schematically depicts a cross-section of an example embodiment of a handpiece 402 for plasma treatment of a periodontal pocket. Handpiece 402 is different from handpiece 302 by being a stand-alone device and having an internal energy source such as a battery. Handpiece 402 is further different from handpiece 302 by employing electromagnetic HV transformation rather than PE transformation to produce a plasma-generating electro-magnetic (EM) field.

Handpiece 402 comprises a proximal part 410 and detachable distal part 320 (the two parts are depicted in FIG. 2D in an attached configuration). Proximal part 410 comprises a battery 430 configured to supply electric power to various electric circuitry in handpiece 402, as is further detailed below. Battery 430 may be a rechargeable battery, adapted to be recharged by a compatible operational unit (not shown).

Proximal part 410 further comprises a HV EM transformer 440. EM transformer 440 is configured to employ EM induction to transform a low alternating voltage at a high current passing through a primary winding 442 to a high voltage at a low current passing through a secondary winding 444, as is well-known in the art. In one or more scenarios, the primary winding and the secondary winding are arranged around a magnetic core 446. Magnetic core 446 may be constructed to form a closed magnetic loop, e.g., as a round or elliptical torus (e.g., shown in FIG. 2D). In some embodiments, magnetic core 446 may be constructed as a rod, forming an open magnetic loop. Magnetic core 446 may be made of magnetic material adapted to the magnetic field strength and field frequency generated by the voltage supplied to the primary windings, as is known in the art. In some embodiments the magnetic core may be made of ferrite and/or from powder iron.

Perhaps to apply a plasma-generating EM field in tip distal portion 132, among other reasons, one terminal of the secondary winding 444 may be electrically connected to pogo-pin contact 452, which may be in electrical contact with HV pogo-pin 366. HV pogo-pin 366 is in electrical contact with anode contact 368 of distal part 320, thereby delivering high voltage to anode conductor 144 (e.g., shown in FIG. 2C). The other terminal of secondary winding 444 may be electrically connected to ground potential.

An alternating voltage may be supplied to the primary windings 442 from an electric circuitry 450. Electric circuitry 450 may receive electric power from the battery 430 and generate an alternating voltage to be supplied to the EM transformer 440. Ground potential may be delivered to hollow tube 140 as described above respective FIG. 2A, namely connecting ground pogo-pin 370 to ground potential of electric circuitry 450.

The alternating voltage's frequency - which may determine the frequency of the EM plasma-generating field at the tip 130 - may be lower than 10MH, typically between 3KHz and 5MHz, in some scenarios between 30KHz and 600KHz. In some embodiments the HV may be modulated ON-OFF, so as to enable simple control over the average power delivered for generating plasma, by controlling the duty cycle of the modulated HV.

In one or more scenarios, the HV EM transformation might not be (e.g., necessarily) related to a standalone device such as handpiece 402, and may be employed in a handpiece that receives energy from an external unit such as an operational unit as described above. In other words, some embodiments of a handpiece according to the teachings herein may comprise an EM HV transformer whereas a low alternating voltage may be supplied to the HV transformer from an electric circuitry in the operational unit via an electric cord. In one or more scenarios of the handpiece, the handpiece may comprise the circuitry required for the low voltage generation, whereas electric power, for example at a low DC voltage, may be supplied from a direct voltage power supply in the operational unit. In one or more scenarios, a handpiece comprising a PE transformer may be adapted as a standalone device, perhaps having a battery as an energy source, instead of being electrically connected to an operational unit, for example.

Proximal part 410 further comprises a gas pump 460 for enforcing gas flow via tip 130 and out from openings 150 in tip distal portion 132. Pump 460 may pump air from the ambient via a gas channel 462, and force the gas via an internal gallery 464 around EM transformer 440 and circuitry 450, into pogo-pin contact channels 466. When distal part 320 is attached to proximal part 410 as depicted in FIG. 2D, pogo-pin contact channels 466 are in flow communication with anode contact perforations 396 hence the gas that passes through anode contact perforations 396 is collected by funnel construction 398 in distal body member 382 and is driven on into hollow tube 140 as described herein.

In one or more scenarios, it may be useful to monitor a plasma parameter and/or a plasma-related parameter. A plasma parameter may include, but is not limited to, plasma glow light intensity, plasma glow light spectrum, and/or spectral components (e.g., the light spectrum in a spectral range or the intensity of a specific spectral line). A plasma related parameter may include a temperature reading perhaps for example near where plasma is generated, the high voltage used to generate the plasma, the low voltage which is transformed to the high voltage, the low-voltage or high-voltage currents, the electric power consumed for plasma generation, and/or a phase difference between two signals (e.g., including between a current and a voltage) in the electric circuit used to generate the high voltage, etc. Such monitoring may be employed, for example as described herein, to verify validity of plasma generation and/or to detect a flaw in the operation of the device. For example, in one or more scenarios, such monitoring may be employed to detect arcing between the anode and the cathode. Such arcing might indicate a breakdown of the insulation between the anode and the cathode and/or might, in one or more scenarios, be accompanied with a (e.g., much more) volatile plasma generation, perhaps as compared to the dielectric-barrier discharge (DBD) mode of operation described herein. As described herein, "arcing" refers to non-DBD discharge between the anode and the cathode, perhaps for example as a result of breakdown of the insulation therebetween, unless otherwise specified, among other scenarios.

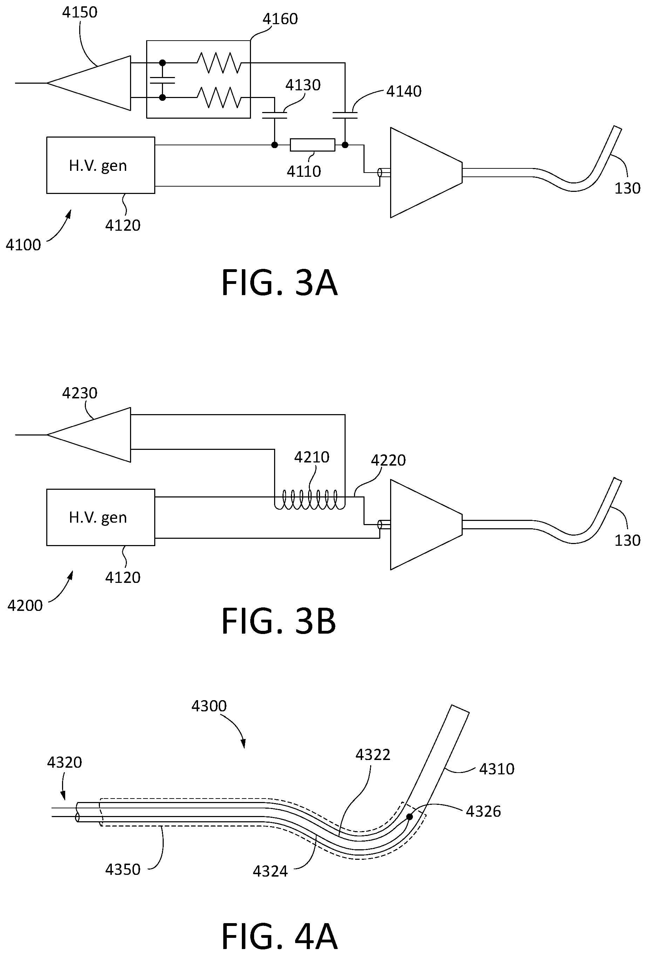

FIG. 3A illustrates schematically an embodiment of an electric circuit 4100 configured to sample the anode current at the tip (e.g., tip 130). Sampling the anode current may enable detection of an arcing event. This is because arcing may be accompanied by a significant change of the anode current compared to the current under normal operation. Perhaps as a result of arcing, among other scenarios, the anode current may in some scenarios increase, while in other scenarios, it may decrease. The current may become irregular, for example characterized by abrupt spikes of high current. Thus, the exact nature of the change of anode current may be dependent upon various characteristics of the plasma generating circuit, including the HV power source, as well as on one or more other characteristics of the device. It may be useful to determine the nature of the change of the anode current and/or the various characteristics independently in one or more, or each, case.

Circuitry 4100 may comprise a serial resistor 4110 connected between the output of a HV power source 4120 and the anode (not shown) of the tip 130. The voltage drop on the resistor, which may be proportional to the anode current, may be collected via buffering capacitors 4130 and/or 4140 and/or may be amplified by amplifier 4150. The capacitors 4130 and/or 4140 may buffer the HV component of the resistor voltage. A low-pass filter 4160 may be employed to protect the amplifier from high frequency voltage spikes. The output of amplifier 4150 may be further processed and/or analyzed (e.g., may be fed to a detection circuit to exploit an envelope signal, and/or be analyzed by a processor such as processor 4010 in FIG. 10) perhaps, for example, to determine whether the device operation may be normal or whether at least some arcing may have occurred.

Electric circuit 4200 illustrated in FIG. 3B may be configured to sample the anode current at the tip (e.g., tip 130). Circuit 4200 may comprise a pick-up coil 4210 that may have its windings wrapped around a wire 4220 connected between the output of the HV power source 4120 and the anode (not shown) of the tip 130. Anode current variations may be transformed to a voltage difference between the terminals of the coil 4220, and may be amplified by amplifier 4230. The output of amplifier 4230 may be further processed and/or analyzed (e.g., may be fed to a detection circuit to exploit an envelope signal, and/or be analyzed by a processor such as processor 4010) perhaps, for example, to determine whether the device operation may be normal or whether at least some arcing may have occurred.

Figure 4A illustrates schematically a tip 4300 that may comprise a hollow tube 4310 and/or an anode (not shown) inside, that perhaps for example in one or more scenarios may be similar to tube 140 and/or anode 142, respectively, of tip 130. Tip 4300 may further comprise a thermocouple 4320 that may comprise a first thermocouple conductor 4322 and/or a second thermocouple conductor 4324, that may be joined to one another at a junction 4326. Junction 4326 may be in thermal contact with the tube 4310. This may enable measuring the tube's temperature, among other scenarios. In one or more scenarios, the thermocouple conductors 4322 and/or 4324 may be stretched along the tube 4310, perhaps for example being electrically insulated from one another except at the point of the junction. In one or more scenarios, the thermocouple conductors 4322 and 4324 may be electrically insulated from the hollow tube 4310 along the entire length of the thermocouple conductors or at least along a portion thereof. In one or more scenarios, the thermocouple conductors 4322 and 4324 and the junction 4326 may be physically attached to the tube - but not necessarily forming Galvanic contact therewith - by a protecting layer 4350. Protecting layer 4350 may be dielectric, for example a layer of lacquer and/or a heat shrink tubing. In one or more scenarios, the thermocouple conductors may be attached to the hollow tube 130 by stripes winding, or the like, for example.

In one or more scenarios, the temperature reading may be used to control the power delivered to plasma generation, for example by reducing the duty cycle of the plasma generating HV, perhaps for example when the temperature rises and/or increasing the duty cycle, perhaps for example when the temperature falls. In one or more scenarios, such control may be done automatically. In one or more scenarios, a temperature reading may be fed to a processor of the device, e.g., processor 4010, and/or the processor may command varying the HV according to the temperature reading.

In one or more scenarios, the temperature reading may be used to detect arcing. For example, an (e.g., exceptional) rise in temperature may be interpreted as an indication of a dielectric breakdown, perhaps leading to arcing between the anode and the cathode. An (e.g., exceptional) temperature rise may be detected by the processor (e.g., processor 4010) which may receive temperature readings. The processor may command the HV to stop, thereby stopping plasma generation, among other scenarios.

Plasma generation as described herein may be accompanied with acoustic sound at the frequency of the on-off modulation of the HV. The sound level (e.g., amplitude) may be dependent on the plasma power consumption. Generally, as the plasma power consumption is greater, the louder the sound. In one or more scenarios, arcing between the anode and the cathode may be detected by monitoring the sounds in the vicinity of the tip. In one or more scenarios, an (e.g., exceptional) change of sound level, for example an exceptional rise thereof, may indicate an arcing between the anode and the cathode.

In one or more scenarios, a sensor of sound such as a microphone (not shown), or the like, may be positioned in the handpiece and/or may be configured to collect sounds from the surroundings of the device tip. In an exemplary scenario a microphone may be positioned in the handpiece, close to the region of plasma generation, namely close to the proximal end of the hollow tube of the tip. The sound reading at the microphone output (output signal of the microphone) may be fed to the device processor (e.g., processor 4010) that may be configured to analyze the microphone output signal and/or may provide operational commands to one or more other device components and/or modules accordingly. In one or more scenarios an output signal of the microphone may be band-pass filtered at a frequency of the pulse modulation of the HV generating the plasma, so that noise at other frequencies (e.g. noise from the surroundings) are filtered out electronically. In one or more scenarios, the processor may command, for example, to stop HV generation, perhaps as a response to a microphone output signal which may be interpreted as indicating arcing between anode and cathode, among other scenarios. In an exemplary scenario an increase in sound level compared to a prior average noise level may indicate arcing, In an exemplary scenario the microphone output signal may be integrated and/or averaged - e.g. over a given time window, for example over a time window of 1-3 seconds - to improve signal to noise ratio and hence improve the validity of arcing detection.

In one or more scenarios, detection of arcing may be carried out off-line, that is to say not during treatment when plasma is activated in a patient's mouth, but rather before and/or after treatment, and/or in-between treatments. Generally, arcing between the anode and the cathode might not expose the patient to risk, harm, and/or danger. Its major drawback may be uncontrolled and/or volatile plasma generation. In one or more scenarios, a check/test for arcing before and/or after each treatment and/or not during treatment may be useful. Perhaps following such a check/test, (e.g., in a rare case of faulty operation) if arcing may be (e.g., actually) detected, correction measures may be taken. For example, the tip may be replaced and/or treatment may be initialized with a validated device, among other correction measures.

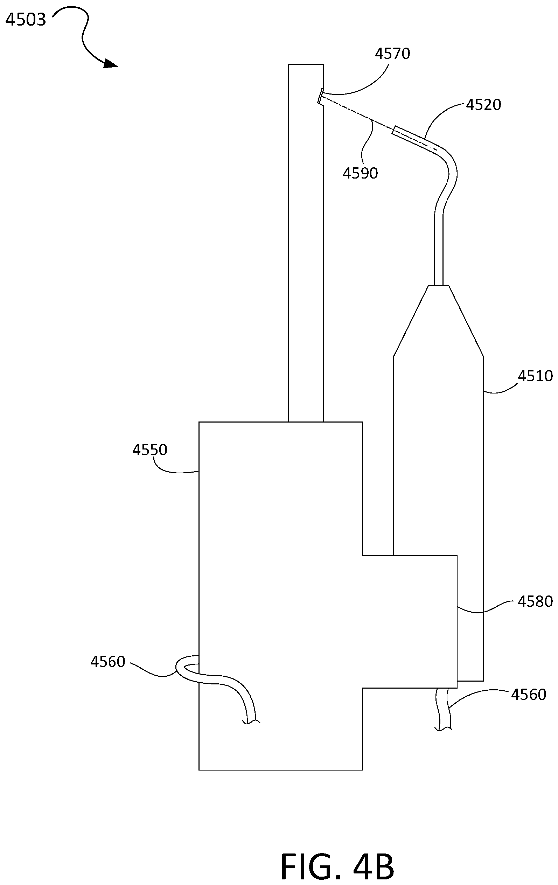

In one or more scenarios, proper plasma generation in DBD mode may be tested off-line. In one or more scenarios, a visual inspection may be used to validate plasma generation. FIG. 4B schematically illustrates an example of a periodontal treatment device 4503. The device 4503 may comprise a handpiece 4510 that may comprise a tip 4520 and/or a base station 4550. The handpiece 4510 may be connected to the base station 4550 by an electric cord and/or a gas tubing 4560, as is described herein regarding device 100 and device 300. In one or more scenarios, the handpiece 4510 may stand alone, as is described herein regarding device 200, having no cord association with the base station 4550, so that communication between the base station 4550 and the handpiece 4520 may be carried out wirelessly.

Base station 4550 may comprise a visual sensing component 4570, configured to collect light from a surrounding thereof. In one or more scenarios, a visual sensing component 4570 may be a camera configured to produce an image, (e.g., a CCD camera, camera 4060 in FIG. 10, etc.). In one or more scenarios, the visual sensing component 4570 may be a light detector, configured to measure light intensity. In one or more scenarios, the visual sensing component 4570 may be an analyzing device, for example a spectral analyzer configured to produce a spectral analysis of the collected light.

Base station 4550 may further comprise a slot 4580 that may be configured to firmly house the handpiece 4510 in a pre-defined position and/or orientation. Perhaps when the handpiece is positioned in slot 4580, the distal end of the tip 4520 may be oriented facing visual sensing component 4570, so that, along a line of sight 4590, the visual sensing component 4570 may collect light from within the hollow tube of the tip 4520.

In one or more scenarios, the handpiece 4510 may be situated in the slot 4580 before actual treatment, in between treatments, and/or after treatment. One or more, or each such occasion that the handpiece 4510 may be so situated in the slot 4580, may be used to test the handpiece 4510 for arcing. One or more tests may be carried out (e.g., manually), perhaps following a command given by the practitioner, such as by pressing a button or the like. In one or more scenarios, a test may be effected automatically by a sensor, not shown, (e.g., via a microswitch, etc., not shown), sensing the handpiece 4510 in the slot 4580. During the test, plasma may be activated (e.g., momentarily) and/or light from the glow inside the tube of the tip 4520 may be collected by visual sensing component 4570.

In some scenarios the collected light intensity may be sampled and compared to a predefined threshold signifying proper operation. Such threshold may be set for example by sampling the measured light intensity during normal operation. In some scenarios measured light intensity that is higher than the set threshold may be used to identify arcing. In some scenarios, light intensity signal may be integrated and/or averaged over a pre-defined time window - e.g. 3-5 seconds - to improve signal to noise ratio and decrease likelihood of false detection of arcing.

In some scenarios the collected light may be employed to form an image. The collected image may be analyzed using an image processing algorithm to determine whether the imaged plasma is in a proper glow discharge mode - characterized by a glowing cloud - or a in an arcing mode, characterized by highly localized and high intensity emission.

In some scenarios the collected light may be used to produce a spectrum. By comparing (e.g., by processor 4010) a measured spectrum to a stored spectrum characterizing proper operation, it may be determined whether the collected light may indicate proper plasma generated in a DBD mode, or arcing.

If arcing is detected, the processor may issue a corresponding indication, for example an alarm to the practitioner, and/or may interlock the generation of plasma.

While it may be useful to generate plasma in the tip 4520 in a DBD mode, and/or to prevent arcing, as explained herein, it may be advantageous in some scenarios to generate plasma by arcing between the anode and the cathode. Generating plasma in a DBD mode may have the advantage of stability (e.g., relatively constant current and/or relatively constant power consumption). Further, plasma may be (e.g., typically) generated over large portions of the inner space of the hollow tube (now shown). As compared to plasma generation by arcing, power dissipation might not be localized, and temperature rise may be (e.g., accordingly) more moderate.

In some scenarios, generating plasma by arcing between the anode and the cathode may prove advantageous over generating the plasma in a DBD mode. At least one advantage may be associated with a higher rate of production of free radicals and/or excited species, perhaps as compared to DBD mode. In one or more scenarios, for example, the HV might be low enough to be insufficient for generating any current between the anode and the cathode in the presence of insulation therebetween, namely in DBD mode. The same voltage may be sufficient to produce arcing, perhaps for example if the distance between the anode and the cathode is small enough.

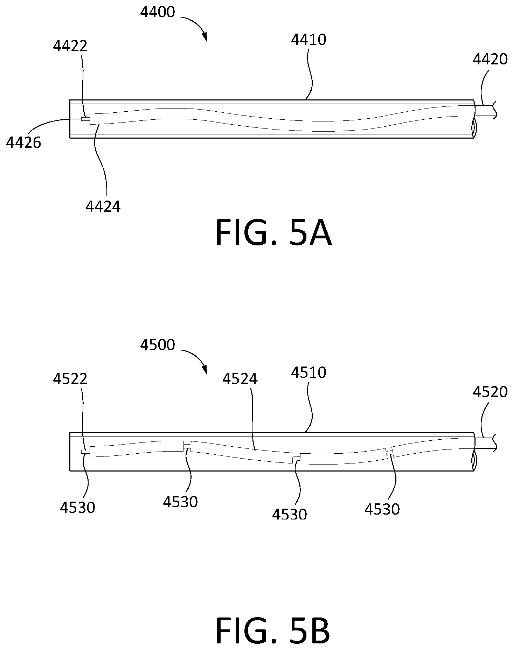

Figure 5A illustrates schematically an example of a tip 4400 that may be configured to produce plasma in an arcing mode. Tip 4400 may comprise a hollow tube 4410 and/or an anode 4420 inside. The anode 4420 may comprise an elongated conductor wire 4422 that may be coated with a dielectric insulation layer 4424. The anode wire 4422 may be electrically associated, in a proximal end thereof, for example, to a HV power source (not shown), as described herein regarding the anode in tip 130. On a distal end 4426, the conductor wire 4422 may be exposed, having no insulation. Perhaps for example in operation, among other scenarios, arcing may be effected between the exposed distal end 4426 and the hollow tube 4410. In one or more scenarios, tip 4400 may also have one or more side holes (not shown).

Figure 5B illustrates schematically an example tip 4500 that may be configured to produce plasma in an arcing mode. Tip 4500 may comprise a hollow tube 4510 and/or an anode 4520 inside. The anode 4520 may comprise an elongated conductor wire 4522 that may be coated, along portions thereof, with a dielectric insulation layer 4524. The anode wire 4522 may be electrically associated, in a proximal end thereof to a HV power source (not shown), as described herein regarding, e.g., tip 4400. The conductor wire 4522 may be exposed in several locations 4530 along a conductor wire 4522 length. In operation, arcing may be effected in one or more exposed locations 4530 which may be closer to an inner wall of the hollow tube 4510, perhaps as compared to the other exposed locations 4530. This may assist in generating plasma in an arcing mode over a distance which may equal or may be close to the thickness of the insulation layer 4524. In one or more scenarios, tip 4500 may also have one or more side holes (not shown).

Atmospheric plasma generation may be (e.g., typically) accompanied by generation of ozone, which is a slowly-decaying toxic gas. When ozone is generated in a patient's mouth, e.g., during a periodontal treatment, the ozone may be (e.g., usefully, preferably, etc.) removed before it is breathed into the patient's lungs. Plasma generating devices according to the teachings herein may generate ozone in the oral cavity creating a concentration of 10PPM in 20 seconds, whereas safety regulations allow ozone concentration of no more than 0.05 ppm in a person's environment.

In one or more scenarios, ozone may be removed from the patient's mouth during plasma-assisted periodontal treatment using a conventional dental suction device. A conventional dental suction device may feature a suction flow rate of above 100 litter/min, perhaps assuring that, if used properly, may remove substantially all the generated ozone, and/or at least may assure maintaining an ozone level lower than the safety level, e.g., below 0.05 ppm or even below 0.001 ppm.

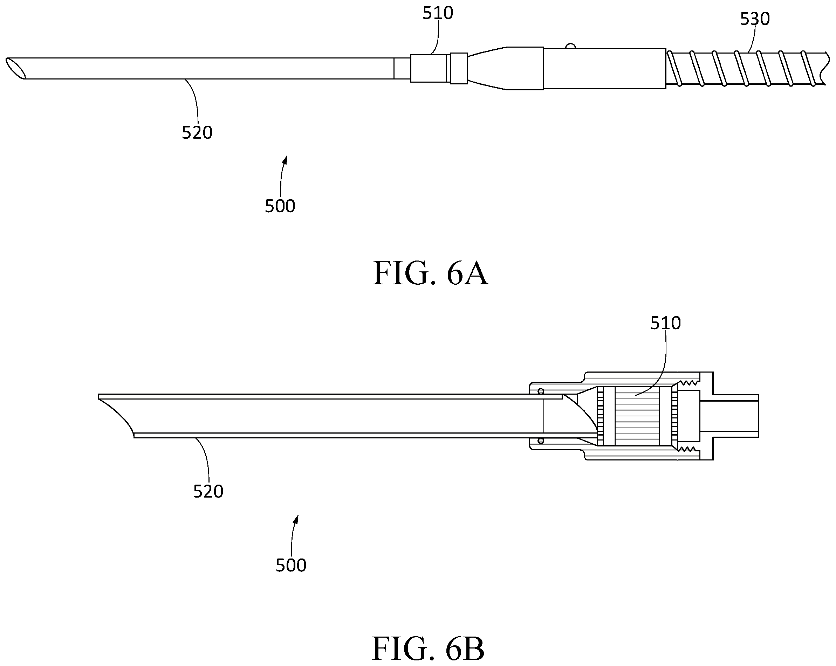

FIG. 6A and FIG. 6B depict schematically an example embodiment of a dental suction device 500 comprising an active filter 510 for absorbing ozone that is sucked by the device. Active filter 510 may be in some embodiments an active carbon filter. FIG. 6A schematically depicts example suction device 500 in side view, illustrating a disposable suction head 520 fluidly associated with a suction tubing 530 via active filter 510. FIG. 6B depicts a cross section of suction head 520 and active filter 510. For use, a kit of disposable components according to the teachings herein for an individual treatment may comprise a distal part for a handpiece, such as distal part 220 or 320, and an active filter such as active filter 510. Before operation, for example, the disposable distal part may be attached to the respective proximal part of the handpiece, whereas the active filter is attached to a suction device as is illustrated in FIG. 6A, between the suction head and the suction tubing.

In one or more scenarios, it may be useful (e.g., necessary) to verify, during plasma operation, that proper suction is maintained in close vicinity to the location where plasma is generated. In other words, it may be desired or necessary, in order to ensure safety and prevent accidental breathing of exceeding quantities of ozone, among other reasons, to stop plasma generation whenever suction is not properly carried out. Thus, according to some embodiments, a plasma-assisted periodontal treatment device according to the teachings herein may be equipped with a pressure sensor at the plasma generating tip or near the plasma generating tip, such that the pressure near the location where plasma is generated may be measured. In one or more scenarios, the pressure measurement by the pressure sensor may be delivered to a controller or a processor of the device, e.g., a controller or a processor in the operational unit of the device. In one or more scenarios, a pressure measurement indicating a pressure lower than the ambient - indicating that suction is properly activated nearby - may allow plasma operation, whereas a pressure measurement near the tip indicating ambient pressure may cause the device' controller to halt plasma generation (e.g., by commanding shutting off EM power supply to the HV transformer of the device, among other interlocks and/or actions).

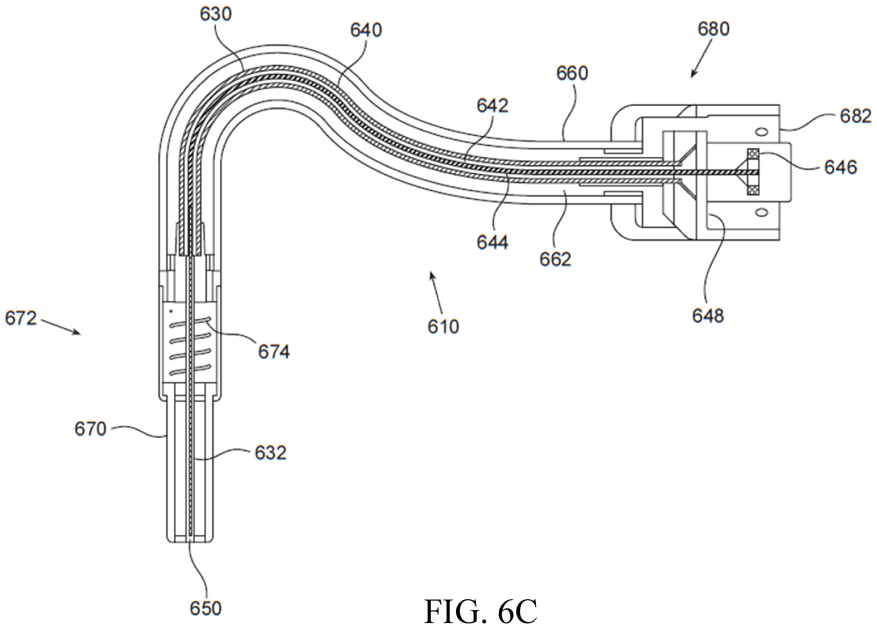

FIG. 6Cschematically depicts an example embodiment of a distal part 610 of a handpiece (not shown) of a plasma-assisted periodontal treatment device. Distal part 610 is different from distal parts 220 or 320 by being configured to suck liquids and/or air from around the plasma generating tip, so as to remove from the patient's mouth plasma residuals such as ozone.

Distal part 610 comprises a plasma generating tip 630 comprising a tip distal portion 632 where during operation plasma may (e.g., usefully, preferably, etc.) be generated. The plasma generating tip may comprise a hollow tube 640 made of an electrically conducting material, for example inert and high-strength metal such as stainless steel. A conducting wire 642 may extend along the hollow tube there inside, and/or may be electrically isolated from the hollow tube, e.g., by an insulation layer 644. During operation, among other scenarios, the conducting wire may receive a high voltage via an anode contact 646 as is explained above regarding anode contact 368, being thereby employed as an anode. Hollow tube 640 may likewise be electrically connected to ground potential via a ground potential contact 648 as is explained and detailed above regarding the previous embodiments. Gas (e.g., air, etc.) may be driven along the hollow tube in a gap between the hollow tube 640 and the conducting wire 642 to be expelled from the hollow tube via openings 650 in tip distal portion 632.

Distal part 610 further comprises a suction tube 660 having a lumen 662 in which hollow tube 640 is arranged concentrically, extending along the suction tube. Suction tube 660 is fluidly associated with a retracting cylinder 670 at a suction distal portion 672 thereof arranged generally around tip distal portion 632. Retracting cylinder 670 is supported by a spring 674 and configured to retract into the suction tube when the tip distal portion 632 is advanced into the periodontal pocket. At a proximal end 680 thereof, distal part 610 comprises a suction channel port 682, which is in fluid communication with the lumen 662 of the suction tube. When the distal part 610 is attached to a corresponding proximal part, among other scenarios, the suction channel port 682 is fluidly associated with a suction channel (not shown) of the proximal part, which is, in turn, fluidly associated with a suction pump (not shown) in the proximal part of the handpiece or in the operation unit to which the handpiece is fluidly associated.

The suction pump may be operative to suck fluids such as gas (e.g., ozone), air, saliva and/or other liquids, via the suction tube 660 and the suction channel port 682, and via the suction channel in the proximal part of the handpiece, thereby removing the ozone from the plasma generation region in the patient's mouth. In some embodiments an exit port (not shown here) of the suction pump may be equipped with an active filter, to prevent release of ozone or other plasma-generated gaseous residuals into the ambient.

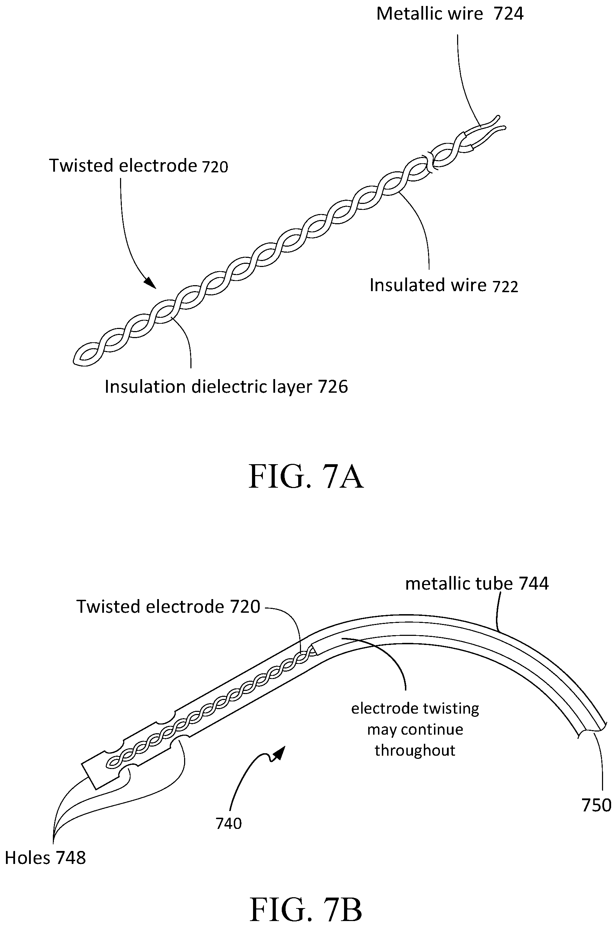

In one or more scenarios, the electrode may take different forms. FIG. 7A illustrates an example twisted electrode 720 that may be used with any of the periodontal treatment devices described herein. The twisted electrode 720 may comprise an insulated wire 722. The insulated wire 722 may comprise a metallic wire 724 having an insulation dielectric layer 726. The insulation layer 726 may be made of a ceramic material or a polymeric material such as, e.g., ETFE and/or PTFE and/or Parylene, among other materials, and having a thickness as is detailed above for FIG. 2B and FIG. 2C. Metallic wire 724 (e.g., stainless steel, etc.) may be exposed at a proximal end thereof, for example for allowing electric contact to the twisted electrode 720. Twisted electrode 720 may have at least the advantage of being manufacturable from a piece of wire that may be cut from a wire roll, or the like.

FIG. 7B illustrates an example configuration 740 of a twisted electrode 720 disposed inside a metallic tube 744 that may be used with any of the periodontal treatment devices described herein. A distal end/part in the metallic tube 744 may comprise one or more holes 748. At 750, the twisted electrode 720 may extend along the at least a part and/or the entirety of the metallic tube 744 so that (e.g., only) the exposed ends may extend out (not shown) of the metallic tube's 744 proximal end/part. The twisting of the twisted electrode 720 may extend along at least a part of, and/or the entire length, of the metallic tube 744. In one or more scenarios, the insulated wire 722 of the twisted electrode 720 may be not twisted at all, where the paired parts of the wire 722 may be lying freely side by side inside the metallic/hollow tube 744.

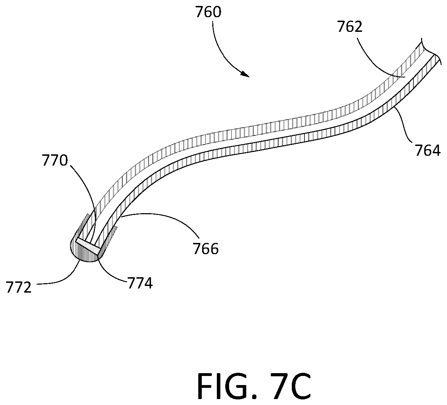

FIG. 7C illustrates an example of a single wire electrode 760 which may be manufacturable from a piece of insulated wire cut from a wire roll. Electrode 760 may comprise an electrically conducting wire 762 that may be insulated with a dielectric insulation layer 764. The conducting wire 762 may be made of an inert metal (e.g., stainless steel). The dielectric insulation layer 764 may be made of a ceramic material and/or a polymeric material such as, for example, ETFE and/or PTFE and/or Parylene^{®}, among other materials. The conducting wire 762 may be exposed at a proximal end (not shown) to enable electrical connection to the electrode 760. At a distal end 766 of the electrode 760, the conducting wire 762 may have an edge 770 which may be electrically insulated from the ambient environment. In one or more scenarios, a dielectric cup 772 may be employed to cover the exposed edge and/or to insulate the edge 770 from the surroundings. In one or more scenarios, the cup 772 may be partly filled with an insulating glue 774 such as epoxy or the like, to fill free spaces and/or slits between the cup 772 and the conducting wire 762 and/or the dielectric insulation layer 764. In one or more scenarios, a layer of glue (e.g., epoxy) may be used to insulate edge 770 without a cup (not shown).

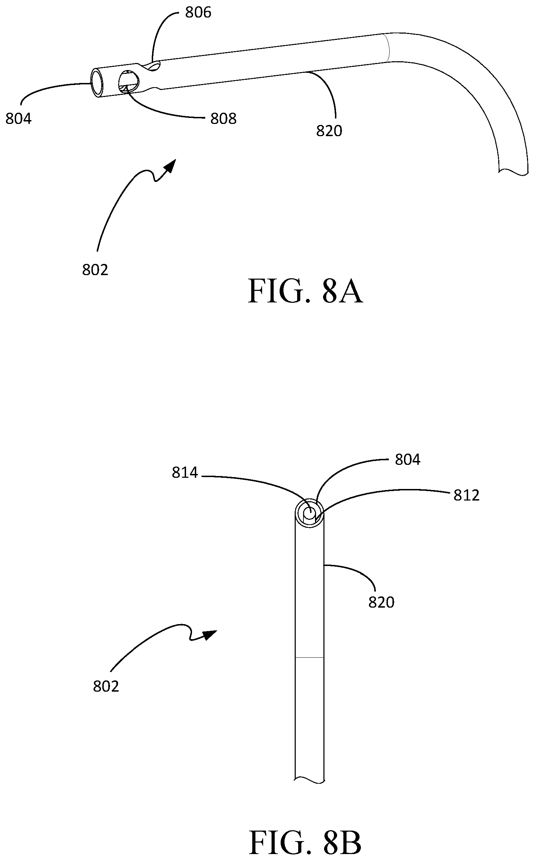

FIG. 8A illustrates an isometric view of an example distal end/part 802 of a metallic tube 820 that may be employed as a cathode according to the teachings herein. Distal end/part 802 may be used with any of the plasma generating treatment devices described herein. The distal end/part 802 may comprise one or more of a distal end hole 804, a pair of first side holes 806, and/or a pair of second side holes 808 in the metallic tube 820.

FIG. 8B illustrates a front view of the distal end/part 802. The distal end/part 802 may comprise a distal end hole 804 in the metallic tube 820. The distal end/part 802 may comprise an electrode 812, that may comprise one or more insulated conductors, such as, for example, anode 142 described herein. The electrode 812 may be disposed in a (e.g., substantially) coaxial and/or concentric configuration in the metallic tube 820. In one or more scenarios, "substantially coaxial" means that the electrode may extend along the long axis of the metallic tube, but not necessarily exactly on the axis itself (e.g., not necessarily in the middle of the tube). Electrode 812 has an electrode distal end 814, that in one or more scenarios may coincide with the distal end of the metallic tube, for example with distal end hole 804.

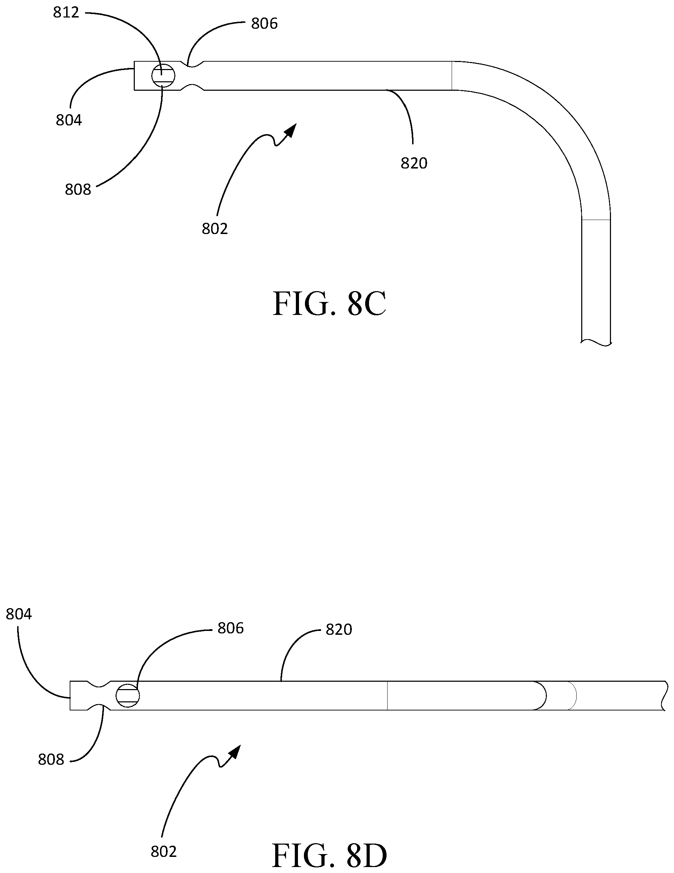

FIG. 8C illustrates a side view of the distal end/part 802 showing holes 804, 806, and/or 808 in the metallic tube 820. The distal end/part 802 may comprise the electrode 812 that may be disposed in a (e.g., substantially) coaxial and/or concentric configuration in the metallic tube 820.

FIG. 8D illustrates a top view of the distal end/part 802 showing holes 804, 806, and/or 808 in the metallic tube 820. The distal end/part 802 may comprise the electrode 812 that may be disposed in a (e.g., substantially) coaxial and/or concentric configuration in the metallic tube 820.

In one or more scenarios, plasma might not be generated properly if liquid is present in the space between the anode and the cathode. In such scenarios, among others, the liquid might form a conductive path for the electric current, thereby shortening the anode to the cathode and preventing generation of the electro-magnetic (EM) field required and/or useful for plasma generation. During treatment, the periodontal pocket might be exposed to mouth fluids such as saliva and/or to rinsing by the medical practitioner, hence devices according to the teachings herein may be configured to prevent and/or reduce risk of shortening the anode high-voltage (HV) by liquid, during operation.

In one or more scenarios, the distal part 802 may be configured so that the electrode distal end 814 is positioned in a location inside the metallic tube 820. In such scenarios, among others, the electrode distal end 814 may be located at a distance of a few millimeters, for example less than 10mm, from the end hole 804. In one or more scenarios, the electrode distal end 814 may be distanced by less than 10mm from the most proximal side hole in the tube (e.g., side hole 806 in FIG. 8A, FIG. 8C, and/or FIG. 8D). In one or more scenarios, distancing the electrode distal end 814 from the holes of the distal part might distance the region of plasma generation from the holes. This might reduce the plasma treatment efficacy. It may be useful to limit and/or to minimize the distance of the electrode distal end 814 from the one or more holes of the metallic tube.

In one or more scenarios, gas flow through the metallic tube, as described herein (e.g., in regard to FIG. 2A and/or FIG. 2D), may assist in preventing liquids from entering the metallic tube. In one or more scenarios, one or more factors may apply to drive liquids into the tube even in the presence of counter gas flow if the flow rate may be too weak. The periodontal pocket might be filled with liquid, hence the tip distal end might be immersed in the liquid to a depth of a few millimeters. Liquid surface tension may further apply a force driving the liquid into the tube through the one or more tube holes. The physical manipulation of the handpiece during treatment and/or temporal rinsing may generate momentary pressure differences, perhaps causing inward momentary fluxes of fluid into the tube, in spite of the outward gas flow, among other factors.

While forced liquid penetration into the tube as described herein may be prevented by a strong enough gas flow, gas injection speed and throughput may not be increased without a limit. An (e.g., overly) strong stream of gas might injure the patient and/or may increase the damage to already-damaged tissues in the periodontal pocket.

In one or more scenarios, perhaps for example by applying gas flow and/or a recessed electrode distal end position, as described herein, plasma generation failure due to liquid penetration into the metallic tube may be prevented, while perhaps maintaining treatment efficacy, for example. Employing gas flow may enable limiting the recession of the electrode distal end by a small-enough distance so that plasma generation may occur on the inner side of the side holes of the metallic tube. Plasma may be generated in (e.g., close) vicinity to the treated region outside the metallic tube. Positioning the electrode distal end at a receded location, perhaps for example a few millimeters away from the distal end of the tube, may enable the use of a (e.g., low enough) gas flow, thus avoiding risk to the treated tissues of the patient. In one or more scenarios, a "trade-off" between the two parameters may exist. In other words, a higher gas flow rate may enable receding the electrode distal end to a smaller distance, and/or vice versa. In other words, receding the electrode distal end to a larger distance from the tube's end may allow employment of gas flow at a lower flow rate. For example, at least one kind of trade off/balance may be obtained with a recession of the electrode by 3 mm from the metallic tube's end, and gas flow rate of 30 ccm, perhaps for example when using a metallic tube having an internal diameter of 0.8mm.

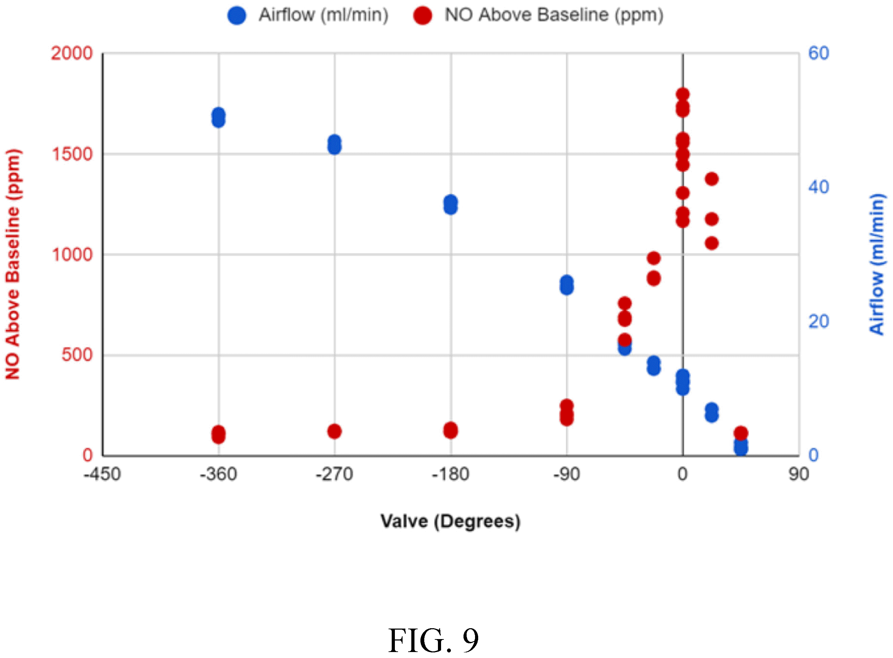

FIG. 9 illustrates an example characteristic of a flow rate of efficacious nitric oxide (NO) output as compared to airflow for one or more of the devices described herein. The characteristic shows an example optimized flow rate of any of the devices described herein, perhaps for example to meet clinician safety requirements while also assessing nitric oxide (NO) output to maintain efficacy using Selected Ion Flow Tube Mass Spectrometry (SIFT-MS). Such data may support efficacious yet low-flow plasma in cold plasma treatment for subgingival applications, among other scenarios, for example.

FIG. 10 is a block diagram of a hardware configuration of an example device that may function as a process control device/logic controller, such as the operational unit 104 of FIG. 1A and/or the operational unit 204 of FIG. 1B, for example. Hardware configuration 4000 may control a user interface such as a graphical user interface to allow a user commanding the device and/or to receive information from the device. Hardware configuration 4000 may collect a measurement reading of a plasma parameter or a plasma-related parameter, e.g., low voltage and/or high voltage, electric current, electric power or glow light intensity, gas pressure or gas flow rate. Hardware configuration 4000 may automatically command activating or stopping a function of the device, upon such a measurement reading. For example, hardware configuration 4000 may command stopping plasma generation (e.g., by causing the HV to shut off) due to one or more such plasma-related parameters being out of a pre-determined range. Hardware configuration 4000 may automatically command varying a function of the device, during operation, among other scenarios.

The hardware configuration 4000 may be operable to facilitate delivery of information from an internal server of a device. The hardware configuration 4000 can include a processor 4010, a memory 4020, a storage device 4030, and/or an input/output device 4040. One or more of the components 4010, 4020, 4030, and 4040 can, for example, be interconnected using a system bus 4050. The processor 4010 can process instructions for execution within the hardware configuration 4000. The processor 4010 can be a single-threaded processor or the processor 4010 can be a multi-threaded processor. The processor 4010 can be capable of processing instructions stored in the memory 4020 and/or on the storage device 4030.

The memory 4020 can store information within the hardware configuration 4000. The memory 4020 can be a computer-readable medium (CRM), for example, a non-transitory CRM. The memory 4020 can be a volatile memory unit, and/or can be a non-volatile memory unit.

The storage device 4030 can be capable of providing mass storage for the hardware configuration 4000. The storage device 4030 can be a computer-readable medium (CRM), for example, a non-transitory CRM. The storage device 4030 can, for example, include a hard disk device, an optical disk device, flash memory and/or some other large capacity storage device. The storage device 4030 can be a device external to the hardware configuration 4000.

The input/output device 4040 may provide input/output operations for the hardware configuration 4000. The input/output device 4040 (e.g., a transceiver device) can include one or more of a network interface device (e.g., an Ethernet card), a serial communication device (e.g., an RS-232 port), one or more universal serial bus (USB) interfaces (e.g., a USB 2.0 port) and/or a wireless interface device (e.g., an 802.11 card). The input/output device can include driver devices configured to send communications to, and/or receive communications from one or more networks (not shown). The input/output device 4000 may be in communication with one or more input/output modules (not shown) that may be proximate to the hardware configuration 4000 and/or may be remote from the hardware configuration 4000. The one or more output modules may provide input/output functionality in the digital signal form, discrete signal form, TTL form, analog signal form, serial communication protocol, fieldbus protocol communication and/or other open or proprietary communication protocol, and/or the like.

The camera device 4060 may provide digital video input/output capability for the hardware configuration 4000. The camera device 4060 may communicate with any of the elements of the hardware configuration 4000, perhaps for example via system bus 4050. The camera device 4060 may capture digital images and/or may scan images of various kinds, such as Universal Product Code (UPC) codes and/or Quick Response (QR) codes, for example, among other images as described herein. In one or more scenarios, the camera device 4060 may be the same and/or substantially similar to any of the other camera devices described herein.

The camera device 4060 may include at least one microphone device and/or at least one speaker device. The input/output of the camera device 4060 may include audio signals/packets/components, perhaps for example separate/separable from, or in some (e.g., separable) combination with, the video signals/packets/components the camera device 460.

The camera device 4060 may be in wired and/or wireless communication with the hardware configuration 4000. In one or more scenarios, the camera device 4060 may be external to the hardware configuration 4000. In one or more scenarios, the camera device 4060 may be internal to the hardware configuration 4000.

The subject matter of this disclosure, and components thereof, can be realized by instructions that upon execution cause one or more processing devices to carry out the processes and/or functions described herein. Such instructions can, for example, comprise interpreted instructions, such as script instructions, e.g., JavaScript or ECMAScript instructions, or executable code, and/or other instructions stored in a computer readable medium.

Implementations of the subject matter and/or the functional operations described in this specification and/or the accompanying figures can be provided in digital electronic circuitry, in computer software, firmware, and/or hardware, including the structures disclosed in this specification and their structural equivalents, and/or in combinations of one or more of them. The subject matter described in this specification can be implemented as one or more computer program products, e.g., one or more modules of computer program instructions encoded on a tangible program carrier for execution by, and/or to control the operation of, data processing apparatus.

A computer program (also known as a program, software, software application, script, or code) can be written in any form of programming language, including compiled or interpreted languages, and/or declarative or procedural languages. It can be deployed in any form, including as a stand-alone program or as a module, component, subroutine, and/or other unit suitable for use in a computing environment. A computer program may or might not correspond to a file in a file system. A program can be stored in a portion of a file that holds other programs and/or data (e.g., one or more scripts stored in a markup language document), in a single file dedicated to the program in question, and/or in multiple coordinated files (e.g., files that store one or more modules, sub programs, or portions of code). A computer program can be deployed to be executed on one computer or on multiple computers that may be located at one site or distributed across multiple sites and/or interconnected by a communication network.

The processes and/or logic flows described in this specification and/or in the accompanying figures may be performed by one or more programmable processors executing one or more computer programs to perform functions by operating on input data and/or generating output, thereby tying the process to a particular machine (e.g., a machine programmed to perform the processes described herein). The processes and/or logic flows can also be performed by, and apparatus can also be implemented as, special purpose logic circuitry, e.g., an FPGA (field programmable gate array) and/or an ASIC (application specific integrated circuit).

Computer readable media suitable for storing computer program instructions and/or data may include all forms of non-volatile memory, media and memory devices, including by way of example semiconductor memory devices (e.g., EPROM, EEPROM, and/or flash memory devices); magnetic disks (e.g., internal hard disks or removable disks); magneto optical disks; and/or CD ROM and DVD ROM disks. The processor and/or the memory can be supplemented by, or incorporated in, special purpose logic circuitry.

While this specification and the accompanying figures contain many specific implementation details, these should not be construed as limitations on the scope of any invention and/or of what may be claimed, but rather as descriptions of features that may be specific to described example implementations. Certain features that are described in this specification in the context of separate implementations can also be implemented in combination in perhaps one implementation. Various features that are described in the context of perhaps one implementation can also be implemented in multiple combinations separately or in any suitable sub-combination. Although features may be described above as acting in certain combinations and/or perhaps even (e.g., initially) claimed as such, one or more features from a claimed combination can in some cases be excised from the combination. The claimed combination may be directed to a sub-combination and/or variation of a sub-combination.

While operations may be depicted in the drawings in an order, this should not be understood as requiring that such operations be performed in the particular order shown and/or in sequential order, and/or that all illustrated operations be performed, to achieve useful outcomes. The described program components and/or systems can generally be integrated together in a single software product and/or packaged into multiple software products.

Examples of the subject matter described in this specification have been described. The actions recited in the claims can be performed in a different order and still achieve useful outcomes, unless expressly noted otherwise. For example, the processes depicted in the accompanying figures do not require the particular order shown, and/or sequential order, to achieve useful outcomes. Multitasking and parallel processing may be advantageous in one or more scenarios.

While the present disclosure has been illustrated and described in detail in the drawings and foregoing description, the same is to be considered as illustrative and not restrictive in character, it being understood that only certain examples have been shown and described, and that all changes and modifications that come within the spirit of the present disclosure are desired to be protected.

### Examples:

Example 1 - A dental device may be configured to generate plasma for treatment of periodontal disease in a targeted tissue in a subject's oral cavity. The device may comprise a tip that may be disposed near the distal end of an electrically conductive tube. The tip may be configured to apply a plasma generating electro-magnetic field.

The electrically conductive tube may comprise a proximal end, the distal end, and at least one hole near the distal end of the electrically conductive tube.

The tip may further comprise at least one electrode. The at least one electrode may be disposed within the electrically conductive tube in at least one orientation with the electrically conductive tube. The disposition may be arranged to provide an air gap between the electrically conductive tube and the at least one electrode at least along a portion of the electrically conductive tube. The at least one electrode may comprise at least one layer of insulation that may completely cover the at least one electrode.

The device may further comprise a source of pressurized gas. The pressurized gas may provide a plasma source when subjected to a plasma generating electro-magnetic (EM) field. A plasma generating EM field may be applied at the tip upon supplying the at least one electrode high voltage relative to the electrically conductive tube.

Example 2 - the device of Example 1, wherein the at least one electrode may be configured to operate as an anode. The electrically conductive tube may be configured to operate as a cathode.

Example 3 - the device of any of Example 1 or Example 2, wherein the air gap may provide a channel via which the generated plasma may flow through the electrically conductive tube.

Example 4 - the device of any of Example 1 to Example 3, wherein a distal portion of the electrically conductive tube may comprise one or more holes. The one or more holes may conduct plasma to the targeted tissue.

Example 5 - the device of any of Example 1 to Example 4, that may further comprise a control unit. The control unit may comprise a memory, a communication interface; and/or a processor. The processor may be configured to monitor a pressure measurement inside the subject's oral cavity. The processor may be configured to compare the pressure measurement to a predetermined pressure value. The processor may be configured to cause the plasma generation to stop upon the pressure measurement exceeding the predetermined value. The processor may be configured to permit the plasma generation to continue upon the pressure measurement being substantially equal to the predetermined pressure value, or less than the predetermined pressure value.

Example 6 - the device of Example 5, wherein the control unit is in wireless, and/or wired, communication with the hand-held dental device.

Example 7 - the device of Example 5, wherein the control unit may comprise a pressure sensor configured to measure the pressure inside a subject's oral cavity

Example 8 - the device of any of Example 1 to Example 7, wherein the electrically conductive tube may be constructed of a metallic substrate.

Example 9 - the device of any of Example 1 to Example 8, wherein the at least one electrode may be constructed of a metallic substrate.

Example 10 - the device of any of Example 1 to Example 9, wherein the at least one electrode may be configured in a twisted configuration.

Example 11 - the device of any of Example 1 to Example 10, that may further comprise a high-voltage (HV) transformer. The HV transformer may provide, at least in part, the EM field.

Example 12 - the device of any of Example 1 to Example 10, further comprising a high-voltage (HV) interface via which to receive HV electrical energy. The (HV) electrical energy may provide, at least in part, the EM field.

Example 13 - the device of any of Example 1 to Example 12, that may further comprise at least one of a gas pump, and/or a gas tube via which to receive the pressurized gas from an external source.

Example 14 - the device of any of Example 1 to Example 13, wherein the device may further comprise at least one of a high-voltage (HV) electrical energy source, and/or an electrical interface that may be configured to receive HV electrical energy from an external source.

Example 15 - the device of any of Example 1 to Example 14, wherein the device may further comprise at least one of a piezoelectric (PE) transformer, and/or a high voltage (HV) transformer.

Example 16 - the device of any of Example 1 to Example 15, wherein the tip may be provided in one of a plurality of physical shapes.

Example 17 - the device of any of Example 1 to Example 16, that may further comprise a suction device configured to draw effluent from the subject's oral cavity.

Example 18 - the device of Example 17, wherein the effluent may comprise at least one of liquid, vapor, and/or gas. At least one effluent gas may be ozone.

Example 19 - the device of Example 17, wherein the device may further comprise a vacuum source. The vacuum source may provide the motive pressure differential to draw the effluent from the subject's oral cavity.

Example 20 - the device of Example 18, wherein the suction device may further comprise at least one active filter that may be configured to absorb at least ozone.

Example 21 - the device of any of Example 14 to Example 20, wherein the device may have an operational condition comprising a normal condition, and/or an arcing condition. The processor may be further configured to measure an anode current. The processor may be further configured to measure voltage corresponding to the HV electrical energy source. The processor may be further configured to determine that the device is in the normal condition, and/or the arcing condition based, at least in part, on the anode current and the voltage corresponding to the HV electrical energy source.

Example 22 - the device of any of Example 5 to Example 21, wherein the tip may further comprise a temperature sensor element configured to measure a temperature of the electrically conductive tube. The processor may be further configured to detect the temperature of the electrically conductive tube. The processor may be further configured to determine a rate of change of the temperature of the electrically conductive tube over a period of time. The processor may be configured to determine if the rate of change of the temperature meets or exceeds a predetermined threshold. The processor may be configured to cause the plasma generation to stop upon the temperature rate of change meeting or exceeding the predetermined threshold. The processor may be configured to permit the plasma generation to continue upon the temperature rate of change being less than the predetermined threshold.

Example 23 - the device of Example 22, wherein the processor may be further configured to determine that the device is in the arcing condition upon the temperature rate of change meeting or exceeding the predetermined threshold.

Example 24 - the device of Example 1 to Example 23, wherein the at least one electrode may be further disposed at 3 millimeters (mm) from the distal end of the electrically conductive tube. The pressurized gas may be provided to the at least one electrode at a rate of 30 cubic centimeters per minute (ccm). The electrically conductive tube may have an internal diameter of 0.8 mm.

Example 25 - A method of operating a dental device that may be configured to generate plasma for treatment of periodontal disease in a targeted tissue in a subject's oral cavity. The device may comprise a tip that may be disposed near a distal end of an electrically conductive tube. The tip may be configured to apply a plasma generating electro-magnetic field.

The electrically conductive tube may comprise a proximal end, the distal end, and at least one hole near the distal end of the electrically conductive tube.

The tip may further comprise at least one electrode. The at least one electrode may be disposed within the electrically conductive tube in at least one orientation with the electrically conductive tube. The disposition may be arranged to provide an air gap between the electrically conductive tube and the at least one electrode at least along a portion of the electrically conductive tube. The at least one electrode may comprise at least one layer of insulation that may completely cover the at least one electrode.

The device may further comprise a source of pressurized gas. The pressurized gas may provide a plasma source when subjected to a plasma generating electro-magnetic (EM) field.

The method may comprise applying a flow of the pressurized gas to the at least one electrode via the electrically conductive tube. The method may comprise supplying the at least one electrode with high voltage relative to the electrically conductive tube. The method may comprise applying the plasma generating EM field at the tip. The method may comprise generating plasma proximate to the at least one hole of the electrically conductive tube.

Example 26 - the method of Example 24, that may further comprise monitoring a pressure measurement inside the subject's oral cavity. The method may comprise comparing the pressure measurement to a predetermined pressure value. The method may comprise causing the plasma generation to stop upon the pressure measurement exceeding the predetermined value. The method may comprise permitting the plasma generation to continue upon the pressure measurement being substantially equal to the predetermined pressure value, or less than the predetermined pressure value.

Example 27 - the method of any of Example 25 to Example 26, wherein the device may have an operational condition comprising a normal condition, and/or an arcing condition. The at least one electrode may operate as an anode. The electrically conductive tube may operate as a cathode. The device may electrically communicate with a high-voltage (HV) electrical energy source.

The method may further comprise measuring an anode current. The method may further comprise measuring a voltage corresponding to the HV electrical energy source. The method may further comprise determining that the device is in the normal condition, and/or the arcing condition based, at least in part, on the anode current and the voltage corresponding to the HV electrical energy source.

Example 28 - the method of any of Example 25 to Example 27, wherein the tip may further comprise a temperature sensor element that may be configured to measure a temperature of the electrically conductive tube.

The method may further comprise detecting the temperature of the electrically conductive tube. The method may comprise determining a rate of change of the temperature of the electrically conductive tube over a period of time. The method may comprise determining if the rate of change of the temperature meets or exceeds a predetermined threshold. The method may comprise causing the plasma generation to stop upon the temperature rate of change meeting or exceeding the predetermined threshold. The method comprising permitting the plasma generation to continue upon the temperature rate or change being less than the predetermined threshold.

Example 29 - the method of Example 28, that may further comprise determining that the device is in the arcing condition upon the temperature rate of change meeting or exceeding the predetermined threshold.

Example 30 - the method of any of Example 25 to Example 29, wherein the air gap may provide a channel via which the generated plasma may flow through the electrically conductive tube. The electrically conductive tube may comprise one or more holes. The one or more holes may conduct plasma to the targeted tissue.

Example 31- A device for treatment of periodontal disease in a subject's oral cavity. The device may comprise a handpiece. The handpiece may comprise a proximal part and a distal part. The distal part may comprise a tip. The tip may comprise a hollow tube that may have at least one hole near a distal end thereof. The tip may further comprise an electrode in the hollow tube. The hollow tube and/or the electrode may be electrically conductive and/or electrically isolated from one another.

The device may comprise a source of pressurized gas fluidly associated with the hollow tube. The device may be configured to stream the gas through the hollow tube to expel to the ambient target area/environment through the at least one hole.

The device may comprise an electro-magnetic (EM) high voltage (HV) power source that may be configured to supply to the electrode HV relative to the hollow tube. The device may be configured to apply a plasma-generating EM field between the electrode and the hollow tube.

The device may be configured to generate plasma at the tip upon the electrode being supplied with (e.g., suitable) HV power.

Example 32 - the device of Example 31, wherein the distal part may be detachable from the proximal part.

Example 33 - the device of Example 31, wherein at least one of the source of pressurized gas and/or the EM HV power source may be housed in a desktop unit associated with the handpiece via a cable.

Example 34 - the device of Example 31, wherein the source of pressurized gas and/or the EM HV power source may be housed in the handpiece. The handpiece may further comprise an electric energy source (e.g., a rechargeable battery).

Example 35 - the device of Example 31, wherein the source of pressurized gas may comprise a compressor. The gas may be air from the ambient area/environment.

Example 36 - the device of Example 31, wherein the electrode and the hollow tube may be electrically isolated from one another by a consecutive dielectric layer. Plasma may be generated in the dielectric barrier discharge (DBD) mode.

Example 37 - the device of Example 36, wherein the consecutive dielectric layer may coat the electrode.

Example 38 - the device of Example 36, wherein the consecutive dielectric layer may coat an internal surface of the hollow tube.

Example 39 - the device of Example 31, wherein the electrode and the hollow tube may be electrically isolated from one another by a non-consecutive dielectric layer. Plasma may be generated in an arcing mode.

Example 40 - the device of Example 31, wherein the electrode may comprise a metallic wire extending along the hollow tube there-inside.

Example 41 - the device of Example 31, wherein the EM HV power source may comprise a piezoelectric HV transformer.

Example 42 - the device of Example 31, wherein the EM HV power source may comprise a magnetic step-up transformer.

Example 43 - the device of Example 31, that may further comprise a sensor that may be configured to generate a plasma-related parameter reading upon sensing a plasma-related parameter selected from electric current, electric voltage, electric power, temperature, light intensity, light spectrum, and/or sound.

Example 44 - the device of Example 43, wherein the electric current may comprise a current through the electrode and/or a current proportional thereto. The electric voltage may comprise a voltage between the electrode and the hollow tube and/or a voltage proportional thereto. The electric power may comprise a power consumed by the plasma generation. The temperature may comprise a temperature of the hollow tube. The light may comprise a plasma-related glow. The sound may comprise a sound generated by a plasma discharge.

Example 45 - the device of Example 43, that may further comprise a processor that may be functionally associated with the sensor and/or with the HV power source. The processor may be configured to command activation and/or deactivation of the HV power source upon the plasma related parameter reading received from the sensor meeting and/or exceeding a (e.g., a predetermined) threshold.

Example 46 - A handpiece for treatment of periodontal disease in a subject's oral cavity. The handpiece may comprise a proximal part and a distal part that may be detachable from the proximal part. The distal part may comprise a hollow tube that may have a distal end exposed to the ambient area/environment and/or at least one hole near the distal end. The distal part may further comprise an electrode disposed in the hollow tube. The hollow tube and/or the electrode may be electrically conductive and/or electrically isolated from one another.

The proximal part may comprise a gas channel, a high voltage electric contact, and/or a ground electric contact. The gas channel may be configured to fluidly associate with the hollow tube. The ground contact may be configured to electrically associate with the hollow tube. The HV contact may be configured to electrically associate with the electrode. The distal part may be attached to the proximal part.

Example 47 - the handpiece of Example 46, that may further comprise a compressor fluidly associated with said gas channel.

Example 48 - the handpiece of Example 46, that may further comprise a HV transformer electrically associated with the HV contact.

Example 49 - the handpiece of Example 48, wherein the HV transformer may be a piezoelectric transformer.

Example 50 - the handpiece of Example 48, wherein the HV transformer may be a magnetic step-up transformer.

Example 51 - the handpiece of Example 46, that may further comprise a rechargeable battery.

Example 52 - the handpiece of Example 46, that may further comprise a sensor that may be configured to generate a plasma-related parameter reading upon sensing a plasma-related parameter selected from electric current, electric voltage, electric power, temperature, light intensity, light spectrum, and/or sound.

Example 53 - the handpiece of Example 52, wherein the electric current may comprise a current through the electrode and/or a current proportional thereto. The electric voltage may comprise a voltage between the electrode and the hollow tube and/or a voltage proportional thereto. The electric power may comprise a power consumed by the plasma generation. The temperature may comprise a temperature of the hollow tube. The light may comprise a plasma-related glow. The sound may comprise a sound generated by a plasma discharge.

Example 54 - A distal part that may be attachable to a proximal part of a handpiece for treatment of periodontal disease in a subject's oral cavity. The distal part may comprise a hollow tube that may have a distal end that may be exposed to the ambient area/environment and/or at least one hole near the distal end.

The distal part may comprise an electrode that may be disposed in the hollow tube. The hollow tube and/or the electrode may be electrically conductive and/or electrically isolated from one another.

The distal part may comprise a distal part interface region. The distal part interface region may comprise a distal part gas port fluidly associated with the hollow tube. The distal part gas port may be configured to fluidly associate with a corresponding proximal part gas port of the proximal part for streaming gas from the proximal part into the hollow tube.

The distal part interface region may comprise a distal part ground electric contact that may be electrically associated with the hollow tube. The distal part ground electric contact may be configured to electrically contact with a corresponding proximal part ground electric contact of the proximal part for supplying low voltage from the proximal part to the hollow tube.

The distal part interface region may comprise a distal part HV electric contact that may be electrically associated with the electrode. The distal part HV may be configured to electrically contact with a corresponding proximal part HV electric contact of the proximal part for supplying high voltage from the proximal part to the electrode.

The distal part interface region may comprise a distal part mechanical connector that may be configured to connect to a corresponding proximal part mechanical connector of the proximal part for attaching the distal part to the proximal part.

The distal part interface region may be configured to couple with a corresponding proximal part interface region when the distal part is attached to the proximal part.

Example 55 - the distal part of Example 54, wherein the hollow tube external diameter at the distal end thereof may be less than 2mm and/or less than 1.5mm and/or less than 1mm and/or less than 0.8mm.

Example 56 - the distal part of Example 54, wherein the ground electric contact may be electrically isolated from the HV electric contact up to a voltage of at least 200V and/or at least 500V and/or at least 1KV.
The invention also refers to the following numbered embodiments, wherein the term "claim" refers to "embodiment".
1. A dental device configured to generate plasma for treatment of periodontal disease in a targeted tissue in a subject's oral cavity, the device comprising:
   a tip disposed near the distal end of an electrically conductive tube, the tip configured to apply a plasma generating electro-magnetic field, the electrically conductive tube comprising:
      a proximal end and the distal end, and at least one hole near the distal end of the electrically conductive tube, the tip further comprising:
   at least one electrode, the at least one electrode disposed within the electrically conductive tube in at least one orientation with the electrically conductive tube, the disposition arranged to provide an air gap between the electrically conductive tube and the at least one electrode at least along a portion of the electrically conductive tube, the at least one electrode comprising at least one layer of insulation completely covering the at least one electrode, the device further comprising:
      a source of pressurized gas, the pressurized gas providing a plasma source when subjected to a plasma generating electro-magnetic (EM) field, wherein a plasma generating EM field is applied at the tip upon supplying the at least one electrode high voltage relative to the electrically conductive tube.
2. The device of claim 1, wherein the at least one electrode is configured to operate as an anode, and the electrically conductive tube is configured to operate as a cathode.
3. The device of any of the claim 1 to claim 2, wherein the air gap provides a channel via which the generated plasma may flow through the electrically conductive tube.
4. The device of claim 1 to claim 3, wherein a distal portion of the electrically conductive tube comprises one or more holes, the one or more holes conducting plasma to the targeted tissue.
5. The device of any of the claim 1 to claim 4, further comprising a control unit, the control unit comprising:
   a memory;
   a communication interface; and
   a processor, the processor configured at least to:
      monitor a pressure measurement inside the subject's oral cavity;
      compare the pressure measurement to a predetermined pressure value;
      cause the plasma generation to stop upon the pressure measurement exceeding the predetermined value; and
      permit the plasma generation to continue upon the pressure measurement being at least one of: substantially equal to the predetermined pressure value, or less than the predetermined pressure value.
6. The device of claim 5, wherein the control unit is at least one of: wireless, or wired, communication with the hand-held dental device.
7. The device of claim 5, wherein the control unit comprises a pressure sensor configured to measure the pressure inside a subject's oral cavity
8. The device of any of claim 1 to claim 7, wherein the electrically conductive tube is constructed of a metallic substrate.
9. The device of any of claim 1 to claim 8, wherein the at least one electrode is constructed of a metallic substrate.
10. The device of any of claim 1 to claim 9 wherein the at least one electrode is configured in a twisted configuration.
11. The device of any of claim 1 to claim 10, further comprising a high-voltage (HV) transformer, the HV transformer providing, at least in part, the EM field.
12. The device of any of claim 1 to claim 10, further comprising a high-voltage (HV) interface via which to receive HV electrical energy, the (HV) electrical energy providing, at least in part, the EM field.
13. The device of any of claim 1 to claim 12, further comprising at least one of: a gas pump, or a gas tube via which to receive the pressurized gas from an external source.
14. The device of any of the claims 1 to 13, wherein the device further comprises at least one of: a high-voltage (HV) electrical energy source, or an electrical interface configured to receive HV electrical energy from an external source.
15. The device of any of the claims 1 to 14, wherein the device further comprises at least one of: a piezoelectric (PE) transformer, or a high voltage (HV) transformer.
16. The device of any of the claims 1 to 15, wherein the tip is provided in one of a plurality of physical shapes.
17. The device of any of the claims 1 to 16, further comprising a suction device configured to draw effluent from the subject's oral cavity.
18. The device of claim 17, wherein the effluent comprises at least one of liquid, vapor, or gas, at least one effluent gas being ozone.
19. The device of claim 17, wherein the device further comprises a vacuum source, the vacuum source providing the motive pressure differential to draw the effluent from the subject's oral cavity.
20. The device of claim 18, wherein the suction device further comprises at least one active filter configured to absorb at least ozone.
21. The device of any of claim 14 to claim 20, wherein the device has an operational condition comprising at least one of: a normal condition, or an arcing condition, and the processor is further configured to:
   measure an anode current;
   measure voltage corresponding to the HV electrical energy source; and
   determine that the device is in at least one of: the normal condition, or the arcing condition based, at least in part, on the anode current and the voltage corresponding to the HV electrical energy source.
22. The device of any of claim 5 to claim 21, wherein the tip further comprises a temperature sensor element configured to measure a temperature of the electrically conductive tube, and the processor is further configured to:
   detect the temperature of the electrically conductive tube;
   determine a rate of change of the temperature of the electrically conductive tube over a period of time;
   determine if the rate of change of the temperature meets or exceeds a predetermined threshold;
   cause the plasma generation to stop upon the temperature rate of change meeting or exceeding the predetermined threshold; and
   permit the plasma generation to continue upon the temperature rate of change being less than the predetermined threshold.
23. The device of claim 22, wherein the processor is further configured to determine that the device is in the arcing condition upon the temperature rate of change meeting or exceeding the predetermined threshold.
24. The device of any of claim 1 to claim 23, wherein the at least one electrode is further disposed at 3 millimeters (mm) from the distal end of the electrically conductive tube, and the pressurized gas is provided to the at least one electrode at a rate of 30 cubic centimeters per minute (ccm), the electrically conductive tube having an internal diameter of 0.8 mm.
25. A method of operating a dental device configured to generate plasma for treatment of periodontal disease in a targeted tissue in a subject's oral cavity, the device comprising:
   a tip disposed near a distal end of an electrically conductive tube, the tip configured to apply a plasma generating electro-magnetic field, the electrically conductive tube comprising:
      a proximal end and the distal end, and at least one hole near the distal end of the electrically conductive tube, the tip further comprising:
      at least one electrode, the at least one electrode disposed within the electrically conductive tube in at least one orientation with the electrically conductive tube, the disposition arranged to provide an air gap between the electrically conductive tube and the at least one electrode at least along a portion of the electrically conductive tube, the at least one electrode comprising at least one layer of insulation completely covering the at least one electrode, the device further comprising:
      a source of pressurized gas, the pressurized gas providing a plasma source when subjected to a plasma generating electro-magnetic (EM) field, the method comprising:
   applying a flow of the pressurized gas to the at least one electrode via the electrically conductive tube;
   supplying the at least one electrode with high voltage relative to the electrically conductive tube;
   applying the plasma generating EM field at the tip; and
   generating plasma proximate to the at least one hole of the electrically conductive tube.
26. The method of claim 25, further comprising:
   monitoring a pressure measurement inside the subject's oral cavity;
   comparing the pressure measurement to a predetermined pressure value;
   causing the plasma generation to stop upon the pressure measurement exceeding the predetermined value; and
   permitting the plasma generation to continue upon the pressure measurement being at least one of: substantially equal to the predetermined pressure value, or less than the predetermined pressure value.
27. The method of any of claim 25 to claim 26, wherein the device has an operational condition comprising at least one of: a normal condition, or an arcing condition, the at least one electrode operates as an anode, the electrically conductive tube operates as a cathode, and the device electrically communicates with a high-voltage (HV) electrical energy source, the method further comprising:
   measuring an anode current;
   measuring a voltage corresponding to the HV electrical energy source; and
   determining that the device is in at least one of: the normal condition, or the arcing condition based, at least in part, on the anode current and the voltage corresponding to the HV electrical energy source.
28. The method of any of claim 25 to claim 27, wherein the tip further comprises a temperature sensor element configured to measure a temperature of the electrically conductive tube, the method further comprising:
   detecting the temperature of the electrically conductive tube;
   determining a rate of change of the temperature of the electrically conductive tube over a period of time;
   determining if the rate of change of the temperature meets or exceeds a predetermined threshold;
   causing the plasma generation to stop upon the temperature rate of change meeting or exceeding the predetermined threshold; and
   permitting the plasma generation to continue upon the temperature rate or change being less than the predetermined threshold.
29. The method of claim 28, further comprising determining that the device is in the arcing condition upon the temperature rate of change meeting or exceeding the predetermined threshold.
30. The method of any of claim 25 to claim 29, wherein the air gap provides a channel via which the generated plasma may flow through the electrically conductive tube, and the electrically conductive tube comprises one or more holes, the one or more holes conducting plasma to the targeted tissue.

## Claims

1. A dental device (100) configured to generate plasma for treatment of periodontal disease in a targeted tissue in a subject's oral cavity, the device (100) comprising:
a tip (130) comprising an electrically conductive tube (140), the electrically conductive tube (140) having a proximal end, a distal end, and at least one hole (150) near the distal end;
at least one electrode (142) disposed within the electrically conductive tube (140), the disposition being arranged to provide an air gap between the electrically conductive tube (140) and the at least one electrode (142) at least along a portion of the electrically conductive tube (140), the at least one electrode (142) comprising at least one layer of insulation (146) completely covering the at least one electrode (142);
a source of pressurized gas (460) fluidly associated with the electrically conductive tube (140), the pressurized gas providing a plasma source when subjected to a plasma-generating electro-magnetic (EM) field;
a high-voltage (HV) electrical energy source (4120) configured to supply the at least one electrode (142) high voltage relative to the electrically conductive tube (140), thereby applying the plasma-generating EM field at the tip (130);
at least one sensor configured to generate at least one plasma-related parameter reading upon sensing a plasma-related parameter selected from electric current, electric voltage, electric power, temperature, light intensity, light spectrum, and sound; and
a processor (4010) functionally associated with the at least one sensor and the HV electrical energy source (4120), the processor (4010) configured to determine whether the device (100) is in a normal condition or an arcing condition between the at least one electrode (142) and the electrically conductive tube (140) based, at least in part, on the at least one plasma-related parameter reading.

2. The device (100) of claim 1, wherein the processor (4010) is further configured to:
cause the plasma generation to stop upon determining that the device (100) is in the arcing condition; and
permit the plasma generation to continue upon determining that the device (100) is in the normal condition.

3. The device (100) of any of claims 1 to 2, wherein the at least one electrode (142) is configured to operate as an anode, the electrically conductive tube (140) is configured to operate as a cathode, and the processor (4010) is further configured to:
measure an anode current;
measure voltage corresponding to the HV electrical energy source (4120); and
determine whether the device (100) is in the normal condition or the arcing condition based, at least in part, on the anode current and the voltage corresponding to the HV electrical energy source (4120).

4. The device (100) of claim 3, wherein the at least one sensor comprises a current-sampling circuit (4100) having a serial resistor (4110) connected between an output of the HV electrical energy source (4120) and the at least one electrode (142), a voltage drop across the serial resistor (4110) being proportional to the anode current.

5. The device (100) of claim 4, wherein the current-sampling circuit (4100) further comprises:
at least one buffering capacitor (4130, 4140) configured to buffer an HV component of the voltage drop across the serial resistor (4110);
an amplifier (4150) configured to amplify a signal corresponding to the voltage drop; and
a low-pass filter (4160) configured to protect the amplifier (4150) from high-frequency voltage spikes.

6. The device (100) of claim 3, wherein the at least one sensor comprises a current-sampling circuit (4200) having:
a wire (4220) connected between an output of the HV electrical energy source (4120) and the at least one electrode (142);
a pick-up coil (4210) having windings disposed around the wire (4220), the pick-up coil (4210) being configured to transform variations in the anode current into a voltage difference between terminals of the pick-up coil (4210); and
an amplifier (4230) configured to amplify a signal corresponding to the voltage difference.

7. The device (100) of any of claims 4 to 6, further comprising a detection circuit configured to generate an envelope signal from a signal corresponding to the anode current, wherein the processor (4010) is configured to determine whether the device (100) is in the normal condition or the arcing condition based, at least in part, on the envelope signal.

8. The device (100) of any of claims 1 to 2, wherein the plasma-related parameter comprises a temperature of the electrically conductive tube (140), and wherein the tip (130) further comprises a temperature sensor element (4320) configured to measure the temperature of the electrically conductive tube (140).

9. The device (100) of claim 8, wherein the processor (4010) is further configured to:
detect the temperature of the electrically conductive tube (140);
determine a rate of change of the temperature of the electrically conductive tube (140) over a period of time;
determine whether the rate of change of the temperature meets or exceeds a predetermined threshold;
cause the plasma generation to stop upon the rate of change of the temperature meeting or exceeding the predetermined threshold; and
permit the plasma generation to continue upon the rate of change of the temperature being less than the predetermined threshold.

10. The device (100) of claim 9, wherein the processor (4010) is further configured to determine that the device (100) is in the arcing condition upon the rate of change of the temperature meeting or exceeding the predetermined threshold.

11. The device (100) of any of claims 8 to 10, wherein the temperature sensor element (4320) comprises a thermocouple having:
a first thermocouple conductor (4322);
a second thermocouple conductor (4324); and
a junction (4326) at which the first thermocouple conductor (4322) and the second thermocouple conductor (4324) are joined, the junction (4326) being in thermal contact with the electrically conductive tube (140),
wherein the first and second thermocouple conductors (4322, 4324) are electrically insulated from one another except at the junction (4326), and are electrically insulated from the electrically conductive tube (140) along at least a portion of the first and second thermocouple conductors (4322, 4324).

12. The device (100) of claim 11, wherein:
the first and second thermocouple conductors (4322, 4324) and the junction (4326) are physically attached to the electrically conductive tube (140) by a dielectric protecting layer (4350); and
the HV electrical energy source (4120) is configured to provide an ON-OFF modulated high voltage, the processor (4010) being configured to vary a duty cycle of the high voltage according to the temperature measured by the temperature sensor element (4320).

13. A control unit (4000) for a dental device (100) configured to generate plasma at a tip (130) by an HV electrical energy source (4120) supplying at least one electrode (142), disposed within an electrically conductive tube (140), high voltage relative to the electrically conductive tube (140), the control unit (4000) comprising:
a memory (4020);
a communication interface (4040) configured to receive at least one plasma-related parameter reading from at least one sensor of the dental device (100); and
a processor (4010) configured to:
determine whether the dental device (100) is in a normal condition or an arcing condition between the at least one electrode (142) and the electrically conductive tube (140) based, at least in part, on the at least one plasma-related parameter reading; and
command deactivation of the HV electrical energy source (4120), thereby stopping plasma generation, upon determining that the dental device (100) is in the arcing condition.

14. The control unit (4000) of claim 13, wherein:
the at least one plasma-related parameter reading comprises an anode current and a voltage corresponding to the HV electrical energy source (4120); and
the processor (4010) is configured to determine whether the dental device (100) is in the normal condition or the arcing condition based, at least in part, on the anode current and the voltage corresponding to the HV electrical energy source (4120).

15. The control unit (4000) of claim 13, wherein the at least one plasma-related parameter reading comprises a temperature of the electrically conductive tube (140), and wherein the processor (4010) is configured to:
determine a rate of change of the temperature of the electrically conductive tube (140) over a period of time;
determine whether the rate of change of the temperature meets or exceeds a predetermined threshold;
determine that the dental device (100) is in the arcing condition upon the rate of change of the temperature meeting or exceeding the predetermined threshold;
command deactivation of the HV electrical energy source (4120) upon the rate of change of the temperature meeting or exceeding the predetermined threshold; and
permit the plasma generation to continue upon the rate of change of the temperature being less than the predetermined threshold.
